(19) Europäisches Patentamt
European Patent Office
Office européen des brevets

(11) EP 1 640 040 A1

(12) **DEMANDE DE BREVET EUROPEEN**

(43) Date de publication:
**29.03.2006 Bulletin 2006/13**

(51) Int Cl.:
***A61Q 1/06*** *(2006.01)* ***A61K 8/37*** *(2006.01)*
***A61K 8/58*** *(2006.01)* ***A61K 8/894*** *(2006.01)*

(21) Numéro de dépôt: **05300591.4**

(22) Date de dépôt: **13.07.2005**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **16.07.2004 FR 0451556**

(71) Demandeur: **L'OREAL**
**75008 Paris (FR)**

(72) Inventeurs:
- **Shimizu, Momoko**
**213-0012, KANAGAWA-KEN (JP)**
- **Blin, Xavier**
**75015, PARIS (FR)**

(74) Mandataire: **Le Coupanec, Pascale A.M.P. et al**
**Nony & Associés,**
**3 rue de Penthièvre**
**75008 Paris (FR)**

(54) **Composition cosmétique comprenant un polymère de silicone**

(57) La présente invention se rapporte à une composition cosmétique, de maquillage et/ou de soin destinée à être appliquée sur la peau, les lèvres et/ou les phanères, et présentant notamment une tenue de la couleur améliorée, tout en ayant une brillance satisfaisante voire améliorée, et un confort satisfaisant, voire amélioré.

EP 1 640 040 A1

## Description

La présente invention se rapporte à une composition cosmétique, de maquillage et/ou de soin destinée à être appliquée sur la peau, les lèvres et/ou les phanères, et présentant notamment une tenue de la couleur améliorée, tout en ayant une brillance satisfaisante voire améliorée, et un confort satisfaisant, voire amélioré.

Les compositions cosmétiques visées par la présente invention sont plus particulièrement des produits de maquillage et/ou de soin destinés à être appliqués sur la peau, les lèvres et/ou les phanères, notamment les rouges à lèvres, les baumes à lèvres, les crayons à lèvres, les fonds de teint liquides ou solides, notamment coulés en stick ou en coupelle, les produits anti-cernes et les produits de coloration de la peau, de tatouages éphémères, les produits de maquillage des yeux comme les eye-liners, en particulier sous forme de crayons, et les mascaras, notamment sous forme de pains ou encore de fards à paupière.

D'une manière générale, les compositions cosmétiques se doivent de conférer un effet esthétique lors de l'application, et de maintenir cet effet esthétique au cours du temps. Elles doivent notamment résister aux différents facteurs extérieurs susceptibles de modifier leur effet esthétique, comme la sueur ou les larmes pour un fond de teint, ou la salive pour un rouge à lèvres.

Les compositions cosmétiques, telles que par exemple les rouges à lèvres, ne doivent pas migrer dans les rides ou ridules, ou transférer sur un tissu. Elles doivent également être agréables à appliquer et maintenir une sensation de confort au cours du temps, tout en conservant des propriétés esthétiques satisfaisantes.

Selon une première alternative, la tenue de la couleur d'une composition cosmétique peut être obtenue par addition d'huile volatile dans la formulation de la composition. L'évaporation de l'huile volatile après application de la composition cosmétique sur la peau ou les lèvres, conduit à une diminution de la fraction liquide et à une concentration du dépôt. Cependant, l'évaporation de l'huile volatile entraîne une sensation d'inconfort et une altération des effets de brillance.

Une autre alternative plus particulièrement proposée pour améliorer la tenue de la couleur des compositions cosmétiques, consiste à réaliser des compositions à partir de sels d'acides alginiques hydrosolubles et de colorants aqueux de manière à éviter l'utilisation d'huile volatile, comme décrit dans la demande de brevet JP 5-271034 et le brevet EP 0 585 981. Cependant, de telles compositions comprenant des colorants aqueux présentent le désavantage de marquer fortement la peau.

D'autres solutions ont été également envisagées sur la base de l'utilisation de dérivés non ioniques de silicone afin d'éviter l'utilisation d'huile volatile. De telles compositions comprenant, par exemple, des dérivés polyoxyalkylénés d'organopolysiloxane ont été décrites dans le brevet EP 0 548 694 et dans les demandes de brevet JP 7-025728, JP 7-330547 et JP 8-027274. Des compositions comprenant des dérivés alkylglycéryléther de silicone ont été décrites dans les demandes JP 6-305933 et JP 7-330547. Des compositions à base huileuse et comprenant des dérivés polyglycérylés de silicone ou fluoroalkylpolyglycérylés de silicone ont été proposées dans les demandes JP 6-157236, JP 9-071504 et JP 10-310504. Des compositions comprenant des dérivés alkylglycérols de silicone ont été également décrites dans le brevet EP 0 475 130 et dans les demandes JP 2-844453 et JP 2-587797. D'autres compositions comprenant des dérivés de silicone hydroxylés par des saccharides, du butylène glycol ou du glycérol, ont été décrites dans les demandes JP 5-186596 et JP 6-145023.

Cependant, en présence d'eau, ces compositions forment un gel à la surface de la peau qui conduit à l'apparition d'une sensation d'inconfort au cours du temps.

La présente invention a précisément pour objet de pallier aux inconvénients des compositions cosmétiques discutées précédemment, et de proposer des compositions cosmétiques présentant une tenue sur les matières kératiniques améliorée tout en maintenant ou en améliorant la brillance et/ou le confort.

Au sens de la présente invention, on entend par les termes « matières kératiniques » couvrir la peau, les muqueuses, comme les lèvres, les ongles et les fibres kératiniques, à l'image des cils et des cheveux.

Le Demandeur a trouvé de manière surprenante que l'incorporation dans une composition cosmétique d'au moins un polymère siliconé tel que décrit ci-après permet d'améliorer la tenue, voir le confort tout en maintenant un bon niveau de brillance de ladite composition cosmétique. Ceci est d'autant plus surprenant que, dans l'art antérieur, l'amélioration de la tenue d'une composition se fait au détriment de la brillance et du confort.

Les compositions cosmétiques conformes à la présente invention sont particulièrement avantageuses pour une utilisation sur la peau et les lèvres.

Les inventeurs ont ainsi découvert, de manière inattendue, que l'utilisation d'un polymère de silicone et de formule générale (I) :

$$R^1_a\, R^2_b\, R^3_c\, SiO_{(4-a-b-c)/2} \qquad (I)$$

dans laquelle $R^1$ est notamment un radical alkyle, $R^2$ est représenté par la formule générale (III) :

Q-O-X (III)

dans laquelle Q est un radical hydrocarboné bivalent et X un radical hydrocarboné polyhydroxylé, et $R^3$ est un groupement organosiloxane de formule générale (IV) :

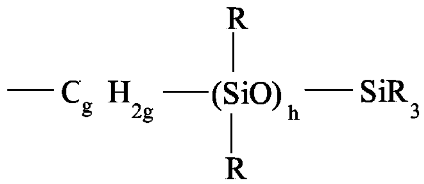

(IV)

dans laquelle chacun des radicaux R représente, indépendamment l'un de l'autre, un radical alkyle, était particulièrement avantageuse pour la formulation de compositions cosmétiques présentant une tenue sur les matières kératiniques améliorée sans nuire à la brillance et au confort du dépôt.

La tenue, la brillance et le confort d'un dépôt de la composition selon l'invention peuvent être évalués selon toute méthode connue de l'homme du métier. Par exemple, la tenue est évaluée comme décrit ci-après.

## TENUE DE LA COMPOSITION

Par « tenue», on entend la propriété de la composition cosmétique selon l'invention à transférer dans une moindre mesure sur des objets avec lesquelles elles peuvent entrer en contact, et la propriété à résister à l'interaction avec des liquides, comme les larmes, la sueur, ou le contact avec des aliments lors d'un repas dans le cas d'un rouge à lèvres par exemple et la propriété à ne pas migrer lors du tracé initial du maquillage, en particulier dans le cas des rouges à lèvres, dans les rides et ridules du contour des lèvres.

La composition cosmétique selon l'invention présente l'avantage de ne pas transférer, du moins en partie, c'est-à-dire de ne laisser que de moindres traces sur certains supports avec lesquels elle peut être mise en contact et notamment un verre, une tasse, une cigarette, un mouchoir, un vêtement ou la peau. Le transfert des compositions cosmétiques provoque une mauvaise tenue du film appliqué, nécessitant de renouveler régulièrement l'application de la composition.

La composition cosmétique selon l'invention présente en outre une bonne tenue de sa couleur. Le film de composition appliqué sur la peau, les lèvres et/ou les phanères peut généralement être altéré lors du contact avec des liquides, notamment de l'eau ou des boissons consommées par exemple lors d'un repas, ou bien encore des huiles comme les huiles alimentaires ou bien encore le sébum ou bien encore de la salive. La tenue de la couleur peut ainsi être caractérisée par la tenue de sa couleur à l'eau et/ou la tenue de sa couleur à l'huile.

Ainsi, l'évaluation des propriétés de tenue de la composition cosmétique selon l'invention peut être caractérisée par la mesure d'au moins l'un des quatre paramètres suivants : le non transfert, la tenue de la couleur à l'eau, la tenue de la couleur à l'huile et la non migration.

On peut mesurer les paramètres de non transfert, de tenue de la couleur à l'eau, et de tenue de la couleur à l'huile les uns à la suite des autres selon le protocole décrit ci-après.

Les mesures sont réalisées, par exemple, sur la face interne de l'avant-bras, lavée et laissée séchée naturellement à température ambiante pendant 5 minutes. La composition cosmétique à tester, par exemple un rouge à lèvres, est appliquée sur trois zones de la face interne de l'avant-bras. La surface de peau sur laquelle les mesures sont réalisées doit être au moins supérieure 1 $cm^2$. De manière générale, les mesures se font sur des zones circulaires de diamètre environ égal à 3 cm.

Il est nécessaire qu'environ la même quantité de composition cosmétique soit appliquée sur chacune des trois zones. Cela peut être vérifié en mesurant le poids de la composition cosmétique, par exemple le rouge à lèvres, après chacune des applications ou en préparant à l'avance des quantités équivalentes d'échantillon à tester. De manière générale, pour une surface de 1 $cm^2$, une quantité égale à environ 2 mg est nécessaire (si la surface possède un diamètre de 3 cm, alors une quantité d'environ 28 mg est requise).

Après application de la composition cosmétique, la couleur, $L_1*a_1*b_1*$, est mesurée en chacune des trois zones, et la valeur moyenne obtenue correspond à la couleur initiale de la composition. La mesure de la couleur peut être effectuée avec un colorimètre MINOLTA de la série CR200 ou CR300 ou CM500 ou CM1000 ou CM2000. On utilise en particulier le colorimètre MINOLTA de la série CR200.

On ajoute 20 $mg/cm^2$ d'eau sur chacune des trois zones à tester (pour des surfaces dont le diamètre est égal à environ 3 cm, environ 280 mg d'eau doivent être appliqués). Chacune des zones à tester est ensuite soumise à un massage manuel pendant quelques secondes, en particulier de 2 à 5 secondes, et plus particulièrement de 2 secondes.

Une épaisseur d'un mouchoir en papier blanc du commerce tel que Kleenex, dont la couleur $L_0*a_0*b_0*$ a été mesurée, est appliqué chaque zone maquillée pendant environ 5 secondes et à une force d'environ 100 g/f, laquelle force peut être appliquée avec un dynamomètre de pression digital DPZ-5N du constructeur IMADA co.ltd.

La valeur de transfert T est obtenue par soustraction de la couleur du tissu blanc mesurée avant application sur la zone à tester, $L_0*a_0*b_0*$, et de la couleur moyenne $L_2*a_2*b_2*$ correspondant à la moyenne des valeurs obtenues pour chaque mouchoir après leur application sur chaque zone de l'avant bras recouverte de composition à tester.

On détermine alors la différence de couleur ΔE(T) entre la couleur du mouchoir avant et après son application sur la zone de l'avant bras portant la composition :

$$\Delta E\ (T) = \sqrt{(L_2^*-L_o^*)^2 + (a_2^* - a_o^*)^2 + (b_2^* - b_o^*)^2}$$

Plus la valeur obtenue ΔE (T) est faible, plus la composition cosmétique est considérée comme présentant un bon niveau de non transfert.

De manière avantageuse, les compositions cosmétiques, selon la présente invention, possèdent une valeur de transfert ΔE (T) comprise entre 0 et 45, et en particulier inférieure ou égale à 45, en particulier inférieure ou égale à 40, notamment inférieure ou égale à 35.

On mesure ensuite la couleur moyenne $L_3*a_3*b_3*$ de la composition après application du mouchoir.

La valeur de tenue de la couleur à l'eau peut être obtenue après réalisation du test de transfert. Elle est par exemple égale à la différence de couleur entre la couleur initiale moyenne $L_1*a_1*b_1*$ de la composition appliquée sur l'avant-bras et de la couleur $L_3*a_3*b_3*$ moyenne de la zone de l'avant-bras portant la composition après application de l'eau et du mouchoir

$$\Delta E\ (E) = \sqrt{(L_3^*-L_1^*)^2 + (a_3^* - a_1^*)^2 + (b_3^* - b_1^*)^2}$$

Plus la valeur obtenue est faible, plus la composition cosmétique est considérée comme possédant une bonne tenue de la couleur à l'eau.

De manière avantageuse, la valeur de tenue de la couleur varie de 0 à 15. Avantageusement, les compositions cosmétiques selon la présente invention, possèdent une valeur de tenue de la couleur à l'eau inférieure ou égale à 15, en particulier inférieure ou égale à 10, et plus particulièrement inférieure ou égale à 6.

Le test de tenue de la couleur à l'huile est conduit par application sur les zones à tester d'environ 20 mg/cm$^2$ d'huile de type alimentaire sur chaque zone de l'avant-bras (huile de colza, huile de soja ou huile de tournesol) suivi d'un massage manuel pendant quelques secondes, en particulier pendant 2 à 5 secondes, et plus particulièrement pendant 2 secondes. Une épaisseur d'un mouchoir en papier blanc du commerce tel qu'un mouchoir Kleenex est ensuite appliquée sur la zone pendant environ 5 secondes et à une force d'environ 100 g/f, laquelle force peut être appliquée avec un dynamomètre de pression digital DPZ-5N du constructeur, IMADA Co.Ltd.

La valeur de tenue de la couleur à l'huile, H, est égale à la différence entre la couleur moyenne $L_4*a_4*b_4*$ de la composition restant sur l'avant bras après le massage à l'huile et l'application du mouchoir, et la couleur moyenne $L_1*a_1*b_1*$ mesurée initialement,

$$\Delta E\ (H) = \sqrt{(L_4^*-L_1^*)^2 + (a_4^* - a_1^*)^2 + (b_4^* - b_1^*)^2}$$

Le test de tenue de la couleur à l'huile est un test permettant notamment d'évaluer le maintien d'une composition cosmétique telle qu'un rouge à lèvres lors d'un repas.

En particulier, le polymère siliconée est tel que, lorsqu'il est en quantité suffisante dans la composition, la tenue de la couleur à l'huile d'un dépôt de ladite composition, une fois étalée sur un support, est inférieure ou égale à 25. De

manière avantageuse, les compositions cosmétiques, selon la présente invention, possèdent une valeur de tenue de la couleur à l'huile inférieure ou égale à 25, en particulier inférieure ou égale à 10, et plus particulièrement inférieure ou égale à 8.

De façon avantageuse, le polymère siliconé est tel que, lorsqu'il est en quantité suffisante dans la composition, la valeur de transfert de ladite composition, une fois étalée sur un support, est inférieure ou égale à 35. La composition a de préférence une valeur de tenue de la couleur à l'eau inférieure à 6, et une valeur de tenue de la couleur à l'huile inférieure ou égale à 8.

## BRILLANCE MOYENNE DE LA COMPOSITION

Avantageusement, le polymère de silicone de formule générale (I) est tel, que lorsqu'il est en quantité suffisante dans la composition cosmétique, la brillance moyenne à 60° d'un dépôt de ladite composition, une fois étalée sur un support, est égale ou supérieure à 30 sur 100.

Par "brillance moyenne", on désigne la brillance telle qu'elle peut être mesurée à l'aide d'un brillancemètre, de manière conventionnelle par la méthode suivante.

Un brillancemètre de type MINOLTA GM268 peut être utilisé. Les mesures sont réalisées sur des zones tests de surface supérieure à 1 $cm^2$. De manière standard, les surfaces sur lesquelles sont étalées les compositions cosmétiques à tester font environ 2,5 cm x 4 cm.

La composition cosmétique à évaluer est appliquée sur une surface synthétique, de type BIOSKIN. La quantité de composition cosmétique à appliquer est d'environ 1 $mg/cm^2$.

La valeur de la brillance est obtenue par mesure de la réflectance à un angle d'environ 60°.

Cinq mesures par échantillon sont nécessaires, la mesure présentant la valeur la plus élevée et la mesure présentant la valeur la plus basse sont écartées, et une moyenne est réalisée sur les trois valeurs mesurées restantes.

La brillance moyenne des compositions cosmétiques selon la présente invention est avantageusement supérieure ou égale à 30, en particulier supérieure ou égale à 40, et plus particulièrement supérieure ou égale à 45.

## CONFORT

Le confort de la composition cosmétique selon l'invention est évalué selon le test décrit ci-après. Dans ce test, le confort de la composition est mesuré par un test de traction d'une bandelette de latex. Ce test prévoit la capacité, pour un dépôt de composition cosmétique, de résister à l'écaillage et au pelage susceptible de survenir à la suite des les mouvements de la peau.

Des échantillons de composition cosmétique sont appliqués sur des surfaces de, par exemple, 2,54 x 2,54 cm, d'une bande de latex de 2,54 cm de large, obtenue, par exemple, par découpe dans la zone du poignet d'un gant de type ANSELL EDMOND INDUSTRIAL TECHNICIANS REF#390, taille 9.

La quantité de composition cosmétique à déposer est telle que le poids en matière sèche de la composition doit être d'environ 20 mg.

La composition cosmétique est appliquée sur la bande de latex à l'aide d'un pinceau à lèvres jetable, par exemple du type de ceux produits par la société FEMME COSMETICS INC., LA.

Les échantillons ainsi préparés sont laissés pendant 24 heures à température ambiante.

Puis, le poids de la bande de latex comprenant le dépôt de composition cosmétique est mesuré (B). La soustraction de la valeur du poids de la bande de latex dépourvue de composition cosmétique (A) de la valeur ainsi mesurée (B) doit correspondre au poids de film sec, et donc être d'environ 20 $\pm$ 2 mg.

La bande de latex portant les échantillons de composition cosmétique à tester est ensuite étirée de telle manière que la zone supportant l'échantillon doit atteindre la longueur d'environ 1,75 pouce (4,445 cm).

Les fragments de film de la composition cosmétique détachés sur la bande de latex sont observés, puis éliminés par balayage à l'aide du pinceau à lèvres.

Le poids de la bande de latex comprenant la composition cosmétique restante, est ensuite mesuré (D).

Le pourcentage de perte de poids du film de la composition cosmétique est ensuite calculé à l'aide de l'équation suivante :

$$\text{Indice de confort} = [(D-A)/(B-A)] \times 100.$$

Les mesures sont répétées trois fois pour chaque composition cosmétique testée. L'indice de confort de la composition selon l'invention est égal à la moyenne de ces trois mesures.

Avantageusement, le polymère de silicone de formule générale (I) est tel, que lorsqu'il est en quantité suffisante

dans la composition cosmétique l'indice de confort d'un dépôt de ladite composition, une fois étalée sur un support, est égale ou supérieure à 90 sur 100, de préférence supérieur à 95 sur 100.

Le Demandeur a également découvert que l'association d'un polymère siliconé de formule (I) et d'une huile siliconée permet d'obtenir une composition cosmétique dont les qualités cosmétiques sont améliorées, en particulier les propriétés de brillance et de tenue sont améliorées.

Ainsi, l'invention, selon un de ses premiers aspects, concerne une composition cosmétique anhydre comprenant dans un milieu physiologiquement acceptable au moins un polymère de silicone de formule générale (I) :

$$R^1_a\ R^2_b\ R^3_c\ SiO_{(4-a-b-c)/2} \qquad (I)$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :
  - les radicaux alkyle en $C_1$-$C_{30}$, le cas échéant substitués par un ou plusieurs atomes de fluor, groupements amino et/ou carboxy,
  - les radicaux aryle, aralkyle, et
  - les radicaux de formule générale (II) :

    $$-C_dH_{2d}-O-(C_2H_4O)_e(C_3H_6O)_fR^4 \qquad (II)$$

    avec :

    - $R^4$ étant un radical en $C_1$-$C_{30}$ ou un radical $R^5$-(CO)- avec $R^5$ étant un radical hydrocarboné en $C_1$-$C_{30}$, et
    - d, e et f étant des entiers tels que d varie de 0 à 15, e et f, indépendamment l'un de l'autre, varient de 0 à 50,

  - et leurs combinaisons,

- $R^2$ est un radical représenté par la formule générale (III) :

  $$-Q-O-X \qquad (III)$$

  avec :

  - Q étant un radical hydrocarboné bivalent en $C_2$-$C_{20}$ pouvant inclure au moins une liaison éther et/ou au moins une liaison ester et
  - X étant un radical hydrocarboné polyhydroxylé,

- $R^3$ est un organosiloxane de formule générale (IV) :

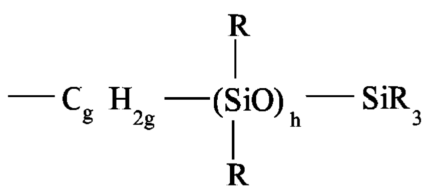

(IV)

avec :

  - chacun des radicaux R représentant un radical alkyle en Ci-$C_{30}$, le cas échéant substitué par un ou plusieurs atomes de fluor, les radicaux aryle, aralkyle,
  - g et h étant des entiers tels que g varie de 1 à 5 et h varie de 0 à 500,

et au moins une huile de silicone.

Selon un second de ses aspects, la présente invention concerne une composition cosmétique anhydre comprenant dans un milieu physiologiquement acceptable au moins un polymère de silicone de formule générale (I), telle

que définie ci-dessus, ledit polymère étant tel que, lorsqu'il est en quantité suffisante dans la composition, la valeur de transfert d'un dépôt de ladite composition, une fois étalée sur un support est inférieure ou égale à 45.

Selon un troisième de ses aspects, la présente invention concerne une composition cosmétique anhydre comprenant dans un milieu physiologiquement acceptable au moins un polymère de silicone de formule générale (I), telle que définie ci-dessus, ledit polymère étant tel que, lorsqu'il est en quantité suffisante dans la composition, la tenue de la couleur à l'eau d'un dépôt de ladite composition, une fois étalée sur un support, est inférieure ou égale à 15.

Selon un quatrième de ses aspects, la présente invention concerne une composition cosmétique anhydre comprenant dans un milieu physiologiquement acceptable au moins un polymère de silicone de formule générale (I), telle que définie ci-dessus, ledit polymère étant tel que, lorsqu'il est en quantité suffisante dans la composition, la tenue de la couleur à l'huile d'un dépôt de ladite composition, une fois étalée sur un support est inférieure ou égale à 25.

Selon encore un cinquième de ses aspects, la présente invention a encore pour objet une composition cosmétique anhydre comprenant dans un milieu physiologiquement acceptable au moins un polymère de silicone de formule générale (I), telle que définie ci-dessus, ledit polymère étant tel que, lorsqu'il est en quantité suffisante dans la composition, la brillance moyenne d'un dépôt de ladite composition, une fois étalée sur un support, est égale ou supérieure à 30.

Selon un sixième de ses aspects, la présente invention concerne encore une composition cosmétique anhydre sous forme coulée comprenant dans un milieu physiologiquement acceptable, au moins un polymère de silicone de formule générale (I), telle que définie ci-dessus.

Selon un septième de ses aspects, la présente invention a pour objet une composition cosmétique anhydre comprenant dans un milieu physiologiquement acceptable au moins un polymère de silicone de formule générale (I), telle que définie ci-dessus, sous réserve que lorsque $R^2$ est un groupement de formule générale (IIIA) :

$$-C_3H_6-O[CH_2CH(OH)CH_2O]_nH \qquad \text{(IIIA)}$$

dans laquelle n est un entier variant de 1 à 5, alors $R^1$ est différent d'un radical alkyle en $C_{12}$.

Selon encore un autre de ses aspects, la présente invention concerne une composition cosmétique telle que définie ci-dessus destinée au maquillage et/ou au soin des lèvres, et/ou de la peau, et en particulier concerne un rouge à lèvres.

Selon encore un autre de ses aspects la présente invention a également pour objet un procédé de maquillage et/ou de soin des matières kératiniques, et notamment de la peau et/ou des lèvres, comprenant l'application sur celles-ci d'au moins une composition cosmétique conforme à la présente invention.

Selon un autre de ses aspects, l'invention a pour objet un support synthétique sur lequel est présent, en tout ou partie de sa surface, au moins une couche d'une composition selon l'invention.

Selon un autre de ses aspects, l'invention a pour objet l'utilisation d'au moins un polymère de silicone de formule générale (I), telle que définie ci-dessus, pour la préparation d'une composition cosmétique anhydre présentant une tenue améliorée associée à une brillance moyenne satisfaisante voire améliorée.

Par « tenue améliorée » on entend une tenue à l'eau améliorée et/ou une tenue à l'huile améliorée et/ou un transfert diminué et/ou une migration diminuée.

Selon encore un autre de ses aspects, l'invention concerne l'utilisation d'au moins un polymère de silicone de formule générale (I), telle que définie ci-dessus, en association avec au moins une huile de silicone pour la préparation d'une composition cosmétique anhydre présentant une tenue améliorée associée à une brillance moyenne satisfaisante, voire améliorée.

Les compositions cosmétiques selon l'invention peuvent se présenter sous forme de pâte, de liquide, de gel, de crème ou de solide. En particulier, les compositions cosmétiques selon l'invention sont de sous forme coulée, et plus particulièrement elles sont sous la forme d'un stick. Elles peuvent être sous la forme d'une émulsion anhydre simple, ou multiple, d'un gel anhydre, solide ou souple.

Par les termes «composition sous forme coulée », on entend, au sens de la présente invention, une composition solide ou semi-solide obtenue à l'issue du refroidissement d'une composition introduite à l'état de fusion, dans un moule. Les compositions peuvent être coulées sous forme d'un stick ou d'un bâton, ou dans une coupelle.

Selon un mode particulier de réalisation, la composition cosmétique selon l'invention est sous forme coulée, c'est-à-dire solide ou semi-solide et plus particulièrement sous la forme d'un stick.

La dureté des compositions coulées selon l'invention peut être mesurée selon la méthode suivante.

Pour déterminer la dureté d'une composition cosmétique conforme à l'invention, on prépare un stick de ladite composition ayant une section circulaire de 12,7 mm de diamètre. Le stick est coulé puis conservé à une température de 20 °C, 24 heures avant de faire la mesure.

La dureté peut être mesurée par la méthode dite « du fil à couper le beurre », qui consiste à couper transversalement le stick à l'aide d'un fil rigide de diamètre 250 µm en tungstène en faisant avancer le fil relativement au stick à une vitesse de 100 mm/min. La dureté correspond à la force maximale de cisaillement exercée par le fil sur le stick à 20 °C, cette force étant mesurée au moyen d'un dynamomètre DFGS2 commercialisé par la société INDELCO-CHATILLON. La dureté est exprimée en grammes.

Selon cette méthode la dureté d'une composition cosmétique conforme à l'invention présentée sous la forme d'un stick varie notamment de 50 à 300 g, en particulier de 70 à 250 g et plus particulièrement de 100 à 230 g.

Un des avantages des compositions cosmétiques selon la présente invention est de présenter une tenue de la couleur améliorée, se traduisant notamment par une migration et/ou un transfert de la couleur réduit(e) et/ou une tenue de la couleur à l'eau améliorée et/ou une tenue de la couleur à l'huile améliorée, et/ou une migration réduite, lors du tracé du maquillage.

Un autre avantage des compositions cosmétiques selon la présente invention est le maintien d'une sensation confortable, d'une absence de sensation collante tout en présentant une bonne adhésion sur la peau et une bonne flexibilité.

Un troisième avantage des compositions cosmétiques selon la présente invention est le maintien de l'effet esthétique, notamment de l'effet de brillance, au cours du temps.

Selon un quatrième avantage, les compositions cosmétiques selon l'invention permettent de conférer une sensation lisse et douce, et de maintenir une bonne hydratation.

Selon un cinquième avantage, les compositions cosmétiques selon la présente invention présentent une bonne tenue face aux facteurs extérieurs susceptibles d'en modifier les propriétés esthétiques, comme la sueur ou un repas, dans le cas d'un rouge à lèvres.

## POLYMÈRE DE SILICONE DE FORMULE GÉNÉRALE (I)

Les polymères de silicone, conformes au polymère de silicone de formule générale (I) et utilisables dans les compositions cosmétiques selon la présente invention, sont décrits en détail dans la demande de brevet EP 1 213 316.

Les polymères de silicone de formule générale (I) présentent l'avantage de pouvoir être utilisés comme agent tensioactif et/ou comme base huileuse.

Dans le cadre de la présente invention, les polymères de silicone de formule générale (I) convenant à la mise en oeuvre des compositions conformes à l'invention sont dénués de fonction d'agent de traitement de surface.

Introduits en quantité suffisante, ils présentent l'avantage de conférer une amélioration de la tenue, voire de la brillance et/ou du confort aux compositions cosmétiques selon l'invention.

En particulier, les polymères de silicone utilisables dans les compositions cosmétiques selon la présente invention sont représentés par la formule générale (I) suivante :

$$R^1_a\ R^2_b\ R^3_c\ SiO_{(4-a-b-c)/2} \qquad (I)$$

dans laquelle :

a) a, b et c sont tels que a varie de 1 à 2,5 ; et b et c, indépendamment l'un de l'autre, varient de 0,001 à 1,5.

b) $R^1$, identique ou différent, est choisi parmi :

- les radicaux alkyles en $C_1$ à $C_{30}$, le cas échéant substitués par un ou plusieurs atomes de fluor, groupements amino et/ou carboxy,
- les radicaux aryle, aralkyle,
- les radicaux de formule générale (II) :

$$-C_dH_{2d}-O-(C_2H_4O)_e(C_3H_6O)_fR^4 \qquad (II)$$

  dans laquelle :

  - $R^4$ est un radical hydrocarboné en $C_1$ à $C_{30}$, ou un radical $R^{5-}$(CO)- avec $R^5$ étant un radical hydrocarboné en $C_1$ à $C_{30}$, et
  - d, e et f sont des entiers tels que d varie de 0 à 15, e et f, indépendamment l'un de l'autre, varient de 0 à 50, et

- leurs combinaisons.

c) $R^2$ est représenté par la formule générale suivante (III) :

$$-Q-O-X \qquad (III)$$

dans laquelle :

- Q est un radical hydrocarboné bivalent en $C_2$ à $C_{20}$, pouvant inclure au moins une liaison éther et/ou au moins une liaison ester, et
- X est un radical hydrocarboné polyhydroxylé.

d) $R^3$ est un groupement organosiloxane de formule générale (IV) :

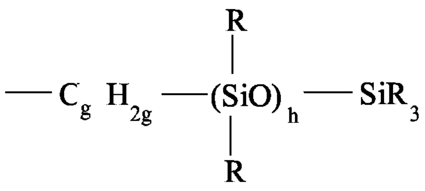

(IV)

avec :

- chacun des radicaux R représentant, indépendamment l'un de l'autre, un radical choisi parmi les radicaux alkyle en $C_1$ à $C_{30}$, le cas échéant substitué par un ou plusieurs atomes de fluor ; et les radicaux aryle et aralkyle,
- g et h étant des entiers tels que g varie de 1 à 5 et h varie de 0 à 500.

Lorsque les radicaux R représentent un radical choisi parmi les radicaux alkyle en $C_1$ à $C_{30}$, le cas échéant substitués par un ou plusieurs atomes de fluor, les radicaux aryle et les radicaux aralkyle, ils ont la même signification que le radical $R^1$ tel que défini précédemment.
Il est à noter que les radicaux $R^1$, $R^2$ et $R^3$ des polymères de silicone de formule générale (I), telle que définie ci-dessus, sont distribués de manière aléatoire ou statistique, c'est-à-dire qu'ils apparaissent dans la structure du polymère sans ordre déterminé. De même, $R^1$, $R^2$, et $R^3$ peuvent figurer respectivement des radicaux de nature différente dans un composé de formule générale (I).
Selon un mode de réalisation particulier,
dans a) :

- de manière plus particulière, a varie de 1,2 à 2,3. Et, en particulier, b et c, indépendamment l'un de l'autre, varient de 0,05 à 1.

dans b) :

- lorsque $R^1$ est un radical alkyle, il peut être un radical alkyle en $C_1$ à $C_{30}$, en particulier un radical alkyle en $C_1$ à $C_{25}$, plus particulièrement un radical alkyle en $C_1$ à $C_{20}$, en particulier un radical alkyle en $C_1$ à $C_{10}$, et notamment un radical alkyle en $C_1$ à $C_6$, et en particulier un radical alkyle en $C_1$ à $C_4$. Plus particulièrement, il peut être un radical méthyle, éthyle, n- ou iso-propyle, n- ou iso- ou tert- butyle, pentyle, hexyle, heptyle, octyle, nonyle, décyle, undécyle ou lauryle. Il peut également être un radical cycloalkyle tel qu'un cyclopropyle, un cyclobutyle, cyclopentyle ou un cyclohexyle. Il peut également être un radical alkyle monoinsaturé ou polyinsaturé, linéaire ou ramifié. Il peut également être un radical alkyle substitué par un ou plusieurs atomes de fluor, tel que le trifluoropropyle ou l'heptadécafluorodécyle. Il peut également être un radical alkyle substitué par un ou plusieurs groupements amino, tels que l'amino-2 éthyle, l'amino-3 propyle, le (amino-2 éthyle)amino-3 propyle. Il peut également être un groupe alkyle substitué par un ou plusieurs groupements carboxy, tel que le carboxy-3 propyle.
- $R^1$ peut également être un radical aryle ou aralkyle tel que le radical phényle, le radical tolyle, le radical benzyle et le radical phénéthyle.
- $R^1$ peut également être un groupe organique représenté par la formule générale (II) :

$$—C_dH_{2d}\text{-}O\text{-}(C_2H_4O)_e(C_3H_6O)_fR^4 \qquad \text{(II)}$$

Selon un mode de réalisation particulier, $R^1$ peut être un radical hydroxylé ou un radical issu de la réaction d'addition d'un alkényléther, saturé ou insaturé, ramifié ou linéaire, dans lequel d = 0 et donc de formule :

$$\text{-}O\text{-}(C_2H_4O)_e(C_3H_6O)_fR^4$$

Dans ce cas, lorsque e et f égalent zéro, alors $R^1$ est un groupe alcoxy ayant de 4 à 30 atomes de carbone, par exemple un radical alcoxy inférieur en $C_4$ à $C_{10}$, tel que le butoxy ou le pentoxy ou un radical alcoxy supérieur, en $C_{11}$ à $C_{30}$, tel que l'oléoxy, le stéaroxy, à savoir par exemple, l'alcool cétylique, l'alcool oléique et l'alcool stéarylique,

ou un radical issu d'un acide ou d'un acide gras, tel que l'acide acétique, l'acide lactique, l'acide butyrique, l'acide oléique, l'acide stéarique et l'acide béhénique.

Lorsque e et f sont supérieurs à 1, alors $R^1$ est un radical hydroxyle provenant de la réaction d'addition d'un alkylène oxyde.

Lorsque e et f égalent zéro, il est particulièrement avantageux que d soit égal à 3, 5 ou 11. Dans ce cas, $R^1$, selon la nature du substituant $R^4$, est un radical allyléther, penthényléther ou undécényléther, ou un radical allylstéaryléther, penthénylbéhényléther, ou undécényloléyléther.

Lorsque e ou f sont différents de zéro, un radical alcoxy et un radical ester sont présents *via* un groupement polyoxyalkylène.

Quelque soit e et f, il est particulièrement avantageux que d soit compris dans l'intervalle variant de 3 à 5.

Selon un mode de réalisation, le radical $R^1$ peut être l'un quelconque des radicaux précédemment définis, ou une combinaison de deux ou plusieurs de ces radicaux.

De manière avantageuse, $R^1$ est un radical alkyle choisi parmi le radical méthyle, le radical lauryle, et leurs combinaisons.

Par ailleurs, lorsque $R^1$ représente dans la même formule générale (I) deux ou plusieurs radicaux, par exemple un radical méthyle et un radical lauryle, ces radicaux apparaissent dans la structure de manière aléatoire, et avec une fréquence qui leur est spécifique.

De manière particulière, au moins 50 % des radicaux $R^1$, en particulier au moins 70 % des radicaux $R^1$, et plus particulièrement 100 % des radicaux $R^1$ sont des radicaux méthyle.

dans c) :

- Q peut être en particulier un radical hydrocarboné bivalent choisi parmi : $-(CH_2)_2-$,$-(CH_2)_3-$, $-CH_2CH(CH_3)-CH_2$, $-(CH_2)_4-$, $-(CH_2)_5-$, $-(CH_2)_6-$, - $(CH_2)_7-$, $-(CH_2)_8-$, $-(CH_2)_9-$, $-(CH_2)_{10}-$, $-(CH_2)_{11}-$, $-(CH_2)_2-CH(CH_2CH_2CH_3)-$, $-CH_2-CH(CH_2CH_3)-$, $-(CH_2)_3-O-(CH_2)_2-$, $-(CH_2)_3-O-(CH_2)_2-O-(CH_2)_2-$, $-(CH_2)_3-O-CH_2CH(CH_3)-$ et $-CH_2-CH(CH_3)-COO(CH_2)_2-$.
  De manière avantageuse, Q est un radical bivalent choisi parmi $-(CH_2)_2-$ et - $(CH_2)_3-$.
- X peut être de manière particulière un radical hydrocarboné polyhydroxylé comprenant au moins deux résidus hydroxyles, et en particulier un groupement hydrocarboné choisi parmi les dérivés glycérylés et les dérivés osidiques.

Les résidus glycérols peuvent être des composés ayant les formules suivantes, dans lesquelles Q a la même signification que dans la formule générale (III), et s et t sont des entiers compris dans l'intervalle variant de 1 à 20, en particulier de 1 à 15, en particulier de 1 à 10, et plus particulièrement de 1 à 5.

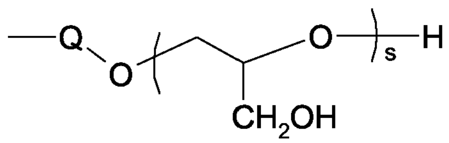

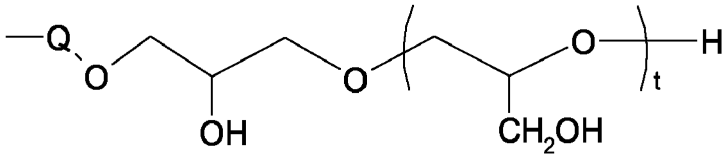

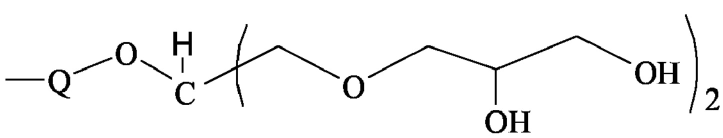

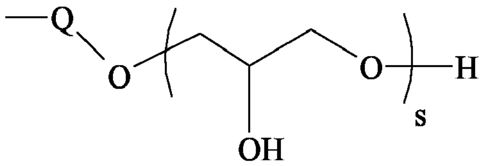

Dans les formules précédentes, un ou plusieurs groupes hydroxyles peuvent être remplacés par des groupes alcoxy ou des groupements esters.
Les radicaux osidiques utilisables dans la formule générale (III) peuvent être de type monosaccharidique, tel que les groupements glycosyle, mannosyle, galactosyle, ribosyle, arabinosyle, xylosyle ou fructosyle, de type oligosaccharidique tel que le maltosyle, le cellobiosyle, le lactosyle ou le maltotriosyle, ou de type polysaccharidique tel que la cellulose ou l'amidon.
De manière particulière, les groupements osidiques sont de type monosaccharidique ou oligosaccharidique. dans d) :

- chacun des radicaux R peut représenter en particulier, indépendamment l'un de l'autre, un radical choisi parmi les radicaux alkyle en de $C_1$ à $C_{20}$, plus particulièrement en $C_1$ à $C_{10}$, en particulier en $C_1$ à $C_6$, le cas échéant substitué par un ou plusieurs atomes de fluor. Lorsque les radicaux R représentent un radical choisi parmi les radicaux alkyle tels que définis précédemment, le cas échéant substitués par un ou plusieurs atomes de fluor, ils ont la même signification que le radical $R^1$ tel que défini précédemment.
- g, selon un mode particulier de réalisation, est égal à 2
- h, selon un mode particulier de réalisation, est compris dans l'intervalle variant de 1 à 50.

Toutefois, parmi les aspects particuliers de la présente invention, définis précédemment, et notamment selon le septième aspect, le polymère de silicone de formule générale (I) est tel que lorsque le radical $R^2$ est représenté par la formule générale (IIIA) :

$$-C_3H_6O[CH_2CH(OH)CH_2O]_nH \qquad \text{(IIIA)}$$

dans laquelle n varie de 1 à 5, alors le radical $R^1$ est différent d'un radical alkyle en $C_{12}$.
Selon un mode particulier de réalisation, le polymère de silicone de formule générale (I) convenant à la mise en oeuvre de la présente invention est tel que :

- a varie de 1 à 1,4, b et c, indépendamment l'un de l'autre, varient de 0,02 à 0,04, et
- $R^1$ est un radical alkyle en $C_1$ à $C_{10}$, en particulier en $C_1$ à $C_6$, et plus particulièrement en $C_1$ à $C_4$.
- $R^2$ est représenté par la formule (IIIA) :

$$C_3H_6O[CH_2CH(OH)CH_2O]_nH \qquad \text{(IIIA)}$$

dans laquelle :

- n varie de 1 à 5, et
- $R^3$ est représenté par la formule (IVA) :

$$-C_2H_4(CH_3)_2SiO[(CH_3)_2SiO]_mSi(CH_3)_3 \qquad \text{(IVA)}$$

dans laquelle :

- m varie de 3 à 9.

Selon un autre mode particulier de réalisation, le polymère de silicone de formule générale (I), utilisable dans les compositions cosmétiques selon la présente invention, est tel que :

- a varie de 1 à 1,4, b et c, indépendamment l'un de l'autre, varient de 0,02 à 0,04,
- $R^1$ est un radical méthyle,
- $R^2$ est représenté par la formule (IIIA) dans laquelle n varie de 1 et 5, et

- $R^3$ est représenté par la formule (IVA) dans laquelle m varie de 3 à 9.

De manière avantageuse, le polymère de silicone de formule générale (I) utilisé dans la composition cosmétique conforme à l'invention, peut être choisi parmi la diméthicone de polyglycéryl-3 polyméthylsiloxyéthyle, la diméthicone de laurylpolyglycéryl-3 polyméthylsiloxyéthyle et la diméthicone de polyglycéryl-3 disiloxane, dont les formules sont respectivement :

- Diméthicone de polyglycéryl-3 polyméthylsiloxyéthyle (formule (V)) :

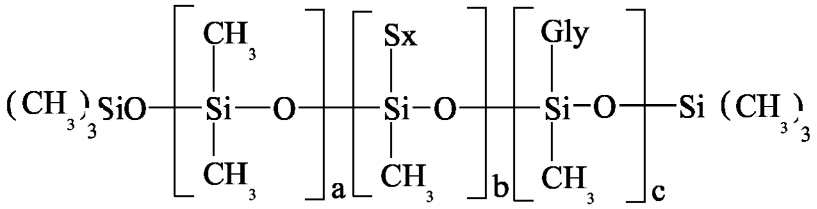

(V)

dans laquelle :

Sx : $-C_2H_4\ [(CH_3)_2SiO]_m si(CH_3)_3$
Gly : $-C_3H_6O[CH_2\text{-}CH(OH)CH_2O]_nH$

avec a = 1-1,4, b = 0,02-0,04, c = 0,02-0,04, m = 3-9, n = 1-5

- Diméthicone de lauryl polyglycéryl-3 polyméthylsiloxyéthyle (formule (VI)) :

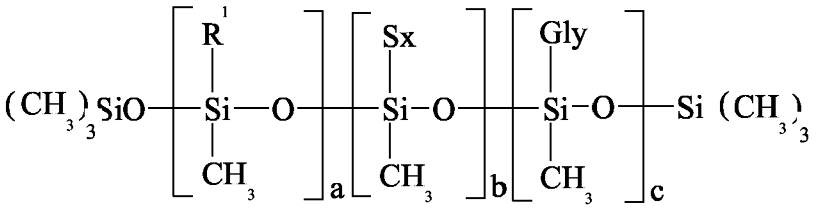

(VI)

dans laquelle Sx, Gly, a, b, c, m et n ont la même signification que précédemment et $R^1$ est, soit un radical méthyle, soit un radical lauryle.

- Diméthicone de polyglycéryl-3 disiloxane (formule (VII)) :

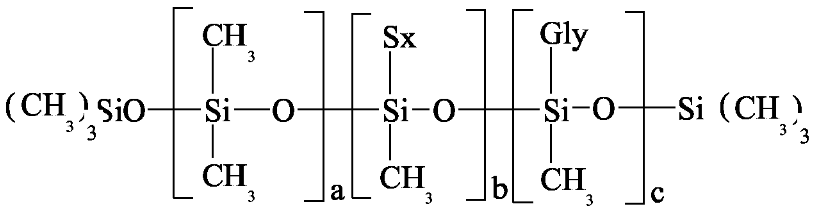

(VII)

dans laquelle Gly, a, b, c, m et n ont la même signification que précédemment, et

Sx : $-O(CH_3)_2SiO\text{-}Si(CH_3)_3$

Le polymère de silicone de formule générale (I) peut être présent dans les compositions cosmétiques conformes à la présente invention à raison de 0,1 à 40 % en poids, en particulier de 0,5 à 30 % en poids, plus particulièrement de 1 à 25 % en poids, en particulier de 5 à 20 % en poids, en particulier de 7 à 15 % en poids, par rapport au poids total de la composition.

Le polymère de silicone de formule générale (I) est, en particulier, mis en oeuvre dans la présente invention sous une forme libre.

Au sens de la présente invention, on entend désigner par "forme libre", une forme du polymère de silicone de formule générale (I) dans laquelle le polymère n'est pas mis en oeuvre sous une forme associée ou adsorbée à un autre matériau, comme décrit par exemple, dans les documents EP 1 416 016 et EP 1 424 373, où il est présent sous la forme d'un enrobage d'une poudre ou d'un agent de coloration afin de bloquer l'activité de surface des particules constituant la poudre correspondante.
Selon un mode particulier de réalisation, le polymère de silicone de formule générale (I) est avantageusement choisi parmi les polymères commercialisés par la société SHIN-ETSU sous les références KF6100® , KF6104® et KF6105® .
Selon encore un autre mode de réalisation, le polymère commercialisé sous la référence KF6104® convient particulièrement à la préparation des compositions cosmétiques conformes à l'invention.
Selon encore un autre mode de réalisation, le composé désigné sous la référence KF6104®, commercialisé par la société SHIN-ETSU, convient particulièrement pour préparer des compositions cosmétiques conformes à l'invention confortables et présentant une tenue sur les matières kératiniques améliorée, sans affectation de la brillance, voire avec une brillance moyenne également améliorée.

**MILIEU PHYSIOLOGIQUEMENT ACCEPTABLE**

Par « milieu physiologiquement acceptable », on désigne un milieu non toxique et susceptible d'être appliqué sur la peau, les lèvres ou matières kératiniques d'êtres humains. Le milieu physiologiquement acceptable est généralement adapté à la nature du support sur lequel doit être appliquée la composition, ainsi qu'à l'aspect sous lequel la composition est destinée à être conditionnée.
Selon une variante, les compositions cosmétiques anhydres conformes à la présente invention peuvent être présentées sous la forme d'une émulsion anhydre, dans laquelle le polymère de silicone de formule générale (I), telle que définie précédemment, peut avoir la fonction de tensioactif.
Au sens de la présente invention, les émulsions contiennent une phase lipophile et une phase hydrophile, cette dernière n'étant pas de l'eau.
Ainsi, les compositions cosmétiques conformes à l'invention peuvent être sous forme anhydre simple ou multiple.
Au sens de la présente invention, on entend par « composition cosmétique anhydre », une composition pouvant contenir moins de 5 %, en particulier moins de 3 %, en particulier moins de 2 %, et plus particulièrement moins de 1 % d'eau par rapport au poids total de la composition. Elles peuvent alors se présenter notamment sous forme de gels huileux, de liquides huileux, de pâtes ou de sticks ou encore sous forme de dispersion vésiculaire contenant des liquides ioniques et/ou non ioniques.

**Phase grasse**

Les compositions cosmétiques conformes à la présente invention peuvent comprendre une phase grasse comprenant notamment des huiles et des corps gras solides à température ambiante (20 - 25 °C) et pression atmosphérique.
On entend par « huile », tout corps gras sous forme liquide à température ambiante (20 - 25 °C) et à pression atmosphérique. La phase grasse liquide peut, également, contenir outre des huiles, d'autres composés solubilisés dans les huiles tels que des agents gélifiants et/ou structurants.
La composition cosmétique selon la présente invention peut comprendre au moins une, et en particulier au moins deux huiles.La ou les huiles peuvent être présentes à raison de 0,1 à 99 % en poids, en particulier d'au moins 1 à 90 % en poids, plus particulièrement de 5 à 70 % en poids, notamment de 10 à 60 % en poids, voire de 20 à 50 % en poids, par rapport au poids total de la composition cosmétique selon l'invention.
Les huiles convenant à la préparation des compositions cosmétiques selon l'invention peuvent être des huiles volatiles ou non, siliconées ou non.
Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm Hg), et de préférence allant de 1,3 Pa à 13 000 Pa (0,01 à 100 mm Hg), et préférentiellement allant de 1,3 Pa à 1300 Pa (0,01 à 10 mm Hg).
Au sens de la présente invention, on entend par « huile non-volatile », une huile ayant une pression de vapeur inférieure à 0,13 Pa.
Les huiles volatiles ou non volatiles peuvent être des huiles hydrocarbonées notamment d'origine animale ou végétale, des huiles synthétiques, des huiles siliconées, des huiles fluorées, ou leurs mélanges.

Au sens de la présente invention, on entend par « huile siliconée», une huile comprenant au moins un atome de silicium, et notamment au moins un groupe Si-O.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en $C_8$-$C_{16}$ (appelées aussi isoparaffines) comme l'isododécane (encore appelé 2,2,4,4,6-pentaméthylheptane), l'isodécane, l'isohexadécane, et par exemple les huiles vendues sous les noms commerciaux d'ISOPARS® ou de PERMETHYLS®.

Comme huiles de silicones volatiles, on peut aussi utiliser des huiles telles que les huiles de silicones linéaires ou cycliques volatiles, notamment celles ayant une viscosité ≤ 8 centistokes ($8 \times 10^{-6}$ $m^2/s$), et ayant notamment de 2 à 10 atomes de silicium, et en particulier de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment les diméthicones de viscosités égales à 5 ou 6 cSt, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et leurs mélanges.

On peut également utiliser des huiles volatiles fluorées tels que le nonafluorométhoxybutane ou le perfluorométhylcyclopentane, et leurs mélanges.

Selon un mode particulier de réalisation, les compositions cosmétiques selon l'invention comprennent moins de 30 % en poids, et en particulier moins de 15 %, en particulier moins de 10 %, et plus particulièrement moins de 5 %, en poids d'huile volatile, par rapport au poids total de la composition.

Selon un autre mode de réalisation, les compositions cosmétiques selon la présente invention sont exemptes d'huiles volatiles.

La phase grasse des compositions cosmétiques selon la présente invention peut également comprendre au moins une huile non volatile.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :

- les huiles hydrocarbonées d'origine animale,
- les huiles hydrocarbonées d'origine végétale telles que les esters de phytostéaryle, tels que l'oléate de phytostéaryle, l'isostéarate de physostéaryle et le glutanate de lauroyl/octyldodécyle/phytostéaryle (AJINOMOTO, ELDEW PS203), les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées ; ces huiles sont notamment des triglycérides héptanoïques ou octanoïques, les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STEARINERIES DUBOIS ou ceux vendus sous les dénominations MIGLYOL 810®, 812® et 818® par la société DYNAMIT NOBEL,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges, et en particulier le polyisobutène hydrogéné,
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit ≥ 10.

Les esters peuvent être notamment choisis parmi les esters, d'acide gras comme par exemple :

- l'octanoate de cétostéaryle, les esters de l'alcool isopropylique, tels que le myristate d'isopropyle, le palmitate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, octanoates, décanoates ou ricinoléates d'alcools ou de polyalcools comme le dioctanoate de propylène glycol, l'octanoate de cétyle, l'octanoate de tridécyle, le 4-diheptanoate et le palmitate d'éthyle 2-hexyle, le benzoate d'alkyle, le diheptanoate de polyéthylène glycol, le diétyl 2-d'hexanoate de propylèneglycol et leurs mélanges, les benzoates d'alcools en

$C_{12}$ à $C_{15}$, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle ;

- les esters de polyols et les esters du pentaérythritol, comme le tétrahydroxystéarate/tétraisostéarate de dipentaérythritol,
- les esters de dimères diols et dimères diacides tels que les Lusplan DD-DA5® et Lusplan DD-DA7®, commercialisés par la société NIPPON FINE CHEMICAL et décrits dans la demande FR0302809 déposée le 6 mars 2003, dont le contenu est incorporé dans la présente demande par référence.
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges, et
- les carbonates de di-alkyle, les 2 chaînes alkyles pouvant être identiques ou différentes, tel que le dicaprylyl carbonate commercialisé sous la dénomination CETIOL CC® , par COGNIS.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates, les diméthicones ou phényltriméthicones de viscosité inférieure ou égale à 100 cSt, et leurs mélanges.

Selon un mode particulier de réalisation, les compositions cosmétiques conformes à l'invention peuvent également comprendre au moins une huile de silicone choisie parmi les huiles de silicones volatiles, les huiles de silicones non volatiles, et leurs mélanges.

De manière avantageuse, l'huile non volatile présente dans les compositions cosmétiques conformes à la présente invention, est notamment choisie parmi le polyisobutène hydrogéné, l'heptanoate d'isostéaryle, l'isononanoate d'isononyle, l'isononanoate d'isotridécyle, le malate de di-isostéaryle, le tétrahydroxystéarate/tétraisostéarate de dipentaérythritol, le 2-octyldodécanol, et leurs mélanges.

Selon un mode de réalisation particulière, l'huile non volatile présente dans la composition cosmétique conforme à la présente invention est un mélange de polyisobutène hydrogéné, d'heptanoate d'isostéaryle, d'isononanoate d'isononyle, d'isononanoate d'isotridécyle, de malate de di-isostéaryle, de tétrahydroxystéarate/tétraisostéarate de dipentaérythritol et de 2-octyldodécanol.

Les huiles non volatiles peuvent être présentes dans les compositions selon l'invention en une teneur allant de 20 à 99 % en poids, notamment de 30 % à 80 % en poids, et en particulier de 40 % à 80 % en poids, par rapport au poids total de la composition.

Selon un mode particulier de réalisation, lorsque la phase grasse liquide des compositions cosmétiques selon la présente invention est une huile de silicone, celle-ci est présente en une teneur allant de 0 à 90 % en poids, notamment de 0,1 à 80 % en poids, et en particulier de 2 à 80 % en poids, par rapport au poids total de la composition.

Selon un autre mode de réalisation, l'huile de silicone est présente dans les compositions cosmétiques conformes à la présente invention, selon un rapport pondéral, par rapport au polymère de silicone de formule générale (I), variant de 80:1, et en particulier de 60:1, et plus particulièrement de 40:1.

Selon un mode de réalisation particulier, les compositions selon l'invention comprennent avantageusement un composé siliconé fluide non volatil.

La phase grasse liquide peut, le cas échéant, être épaissie, gélifiée ou structurée en y incorporant un agent gélifiant de phase grasse, tel que décrit dans la demande WO 2004/55080 publiée le 1er juillet 2004, dont le contenu est incorporé dans la présente demande par référence.

Les compositions selon l'invention peuvent également comprendre au moins un composé choisi parmi les cires, les corps gras pâteux, et leurs mélanges.

La cire est solide à température ambiante (25 °C), à changement d'état solide/liquide réversible, ayant une température de fusion supérieure à 30 °C pouvant aller jusqu'à 200 °C, une dureté supérieure à 0,5 MPa et présentant à l'état solide une organisation cristalline anisotrope. Elle peut être hydrocarbonée, fluorée et/ou siliconée et être d'origine animale, végétale, minérale ou synthétique. Elle peut être choisie par exemple parmi la cire d'abeille, la cire de Carnauba, la cire de Candelilla, les cires de paraffine, l'huile de ricin hydrogénée, les cires synthétiques comme les cires de polyéthylène (de préférence de poids moléculaire compris entre 400 et 600) ou de Fischer-Tropsch, les cires de silicone comme les alkyl- ou alkoxy-diméthicone ayant de 16 à 45 atomes de carbone, les cérésines ou les ozokérites, comme par exemple les isoparaffines dont le point de fusion est inférieur à 40 °C, tel que l'EMW-0003, commercialisé par la société NIPPON SEIROU, les oligomères d'$\alpha$-oléfine, tel que les polymères PERFORMA V® 825, 103 et 260, commercialisés par la société NEW PHASE TECHNOLOGIES ; les copolymères éthylène-propylène, tel que le PERFORMALENE® EP 700, et les cires microcristallines dont le point de fusion est supérieur à 85 °C, tel que les HI-MIC® 1070, 1080, 1090 et 3080, commercialisées par NIPPON SEIROU, et leurs mélanges.

De manière avantageuse, la cire utilisée dans les compositions cosmétiques conformes à l'invention, est choisie parmi les cires de polyéthylène, la cire de Candelilla, et leurs mélanges.

Selon un mode particulier de réalisation, les compositions cosmétiques selon la présente invention comprennent un mélange de cire de polyéthylène et de cire de Candelilla.

Selon un mode particulier de mise en oeuvre, la ou les cires utilisées dans les compositions cosmétique conformes à la présente invention est ou sont présentes en une teneur variant d'environ 1,5 à environ 20 %, en particulier d'environ 3 à environ 15 %, en particulier d'environ 5 à environ 10 %, et plus particulièrement d'environ 6,5 à environ 8,5 % en poids par rapport au poids total de la composition.

Les compositions cosmétiques conformes à la présente invention peuvent également comprendre au moins un composé pâteux.

Par "pâteux" au sens de la présente invention, on entend un composé gras à changement d'état solide/liquide réversible, et comportant à la température de 23°C une fraction liquide et une fraction solide. Est également considéré comme pâteux, le polylaurate de vinyle.

Le composé pâteux au sens de l'invention présente avantageusement une dureté à 20 °C allant de 0,001 à 0,5 MPa, de préférence de 0,002 à 0,4 MPa.

Parmi les composés pâteux susceptibles d'être utilisés dans la composition selon l'invention, on peut citer les lanolines et les dérivés de lanoline comme les lanolines acétylées, les lanolines oxypropylénées ou le lanolate d'isopropyle, et leurs mélanges. On peut également utiliser des esters d'acides ou d'alcools gras, notamment ceux ayant 20 à 65 atomes de carbone comme le citrate de tri-isostéaryle ou de cétyle ; le propionate d'arachidyle ; le polylaurate de vinyle ; les esters du cholestérol comme les triglycérides d'origine végétale tels que les huiles végétales hydrogénées, les polyesters visqueux et leurs mélanges. Comme triglycéride d'origine végétale, on peut utiliser les dérivés d'huile de ricin hydrogénée, tels que le « THIXINR® » de Rheox.

On peut également citer les polyesters résultant de l'estérification d'un acide carboxylique et d'un ester acide hydroxycarboxylique aliphatique, par exemple, le RISOCAST® DA-L (ester issu de la réaction d'estérification de l'huile de ricin hydrogéné avec de l'acide dilinoléïque dans des proportions de 2 pour 1) et le RISOCAST® DA-H (ester résultant de l'estérification de l'huile de ricin hydrogénée avec de l'acide isostéarique dans des proportions de 4 pour 3) commercialisés par la société japonaise KOKYU ALCOHOL KOGYO.

Comme composé pâteux convenant avantageusement à la formulation des compositions cosmétiques conformes à la présente invention, on peut faire mention des coco-glycérides hydrogénés.

On peut aussi citer les composés pâteux siliconés tels que les polydiméthylsiloxanes (PDMS) de hauts poids moléculaires et en particulier ceux ayant des chaînes pendantes du type alkyle ou alcoxy ayant de 8 à 24 atomes de carbone, et un point de fusion de 20-55°C, comme les stéaryl diméthicones notamment ceux vendus par la société DOW CORNING sous les noms commerciaux de DC2503® et DC25514® et leurs mélanges.

## Phase hydrophile

La composition selon l'invention peut comprendre au moins une phase hydrophile qui peut former la phase continue de la composition anhydre.

La phase hydrophile est essentiellement dépourvue d'eau.

Elle peut comprendre un mélange de solvants organiques miscibles à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, et les aldéhydes en $C_2$-$C_4$.

La phase hydrophile peut être présente à une teneur allant de 0,1 à 40 % en poids, notamment allant de 0,1 à 20 % en poids, et en particulier 0,1 à 10 % en poids, par rapport au poids total de la composition.

## POLYOLS

Selon un mode de réalisation, les compositions cosmétiques conformes à l'invention peuvent également comprendre en outre au moins un polyol ou alcool polyhydrique.

Par "alcool polyhydrique" ou "polyol", il faut comprendre, au sens de la présente invention, toute molécule organique comportant au moins deux groupements hydroxyle libres.

Les alcools polyhydriques convenant avantageusement pour la formulation des compositions cosmétiques selon la présente invention sont ceux présentant notamment de 2 à 20 atomes de carbone, en particulier de 2 à 10 atomes de carbone, et plus particulièrement de 2 à 6 atomes de carbones.

Avantageusement, le polyol peut être par exemple choisi parmi la glycérine, le propylène glycol, le butylène glycol, le pentylène glycol, l'hexylène glycol, le dipropylène glycol, le diéthylène glycol, le sorbitol, l'hydroxypropyl sorbitol, le 1,2,6-hexanetriol ;les éthers de glycol (ayant notamment de 3 à 16 atomes de carbone) tels que les alkyl($C_1$-$C_4$) éther de mono, di- ou tripropylène glycol, les alkyl($C_1$-$C_4$)éthers de mono, di- ou triéthylène glycol ; et leurs mélanges.

**AGENTS FILMOGENES**

De manière avantageuse, les compositions cosmétiques conformes à la présente invention peuvent comprendre au moins un agent filmogène, associé ou non avec au moins un auxiliaire de filmification.

Les agents filmogènes comprennent en particulier des polymères filmogènes.

Par « polymère filmogène », on désigne un polymère apte à former à lui seul ou en présence d'un agent auxiliaire de filmification, un film isolable, notamment continu et adhérant sur un support, notamment sur les matières kératiniques, et de préférence un film cohésif et mieux encore, un film dont la cohésion et les propriétés mécaniques sont telles que ledit film peut être isolé dudit support.

Dans la composition, on peut utiliser un seul polymère filmogène ou un mélange de polymères filmogènes. Ce polymère filmogène peut être choisi parmi le groupe constitué par les polymères synthétiques de type polymères radicalaires, ou polycondensats et les polymères d'origine naturelle, et leurs mélanges, tels que ceux décrits dans la demande FR0450540 déposée le 18 mars 2004 dont le contenu est incorporé dans la présente demande par référence.

Dans les compositions cosmétiques conformes à l'invention, la teneur en agents filmogènes peut varier de 0,1 à 20 % en poids, notamment de 0,5 à 20 % en poids, et en particulier de 1 à 20 % en poids, par rapport au poids total de la composition.

Avantageusement, l'agent filmogène est présent, dans les compositions cosmétiques conformes à l'invention, dans un rapport pondéral par rapport au poids des polymères de silicone de formule générale (I), inférieur à 5:1, en particulier inférieur à 1:1, et plus particulièrement inférieur ou égal à 1:2.

Comme polymères filmogènes convenant particulièrement à l'invention, on peut citer les polymères et copolymères acryliques, les polyuréthannes, les polyesters, les polyamides, les polyurées, les polymères cellulosiques comme la nitrocellulose, et les polymères siliconés.

Il est également avantageux d'utiliser des résines de silicone, qui sont des polymères de polyorganosiloxanes réticulés généralement solubles ou gonflables dans les huiles de silicones.

A titre d'exemple de ces résines de silicone, on peut citer les siloxysilicates, les polysilesquioxanes et les polyméthylsesquioxanes.

A titre d'exemple de résines polyméthylsesquioxanes commercialement disponible, on peut citer celles qui sont commercialisées par la société WACKER sous la référence RESIN MK, tel que la BELSIL PMS MK ; celles commercialisées par la société SHIN-ETSU, par exemple sous les références KR-220L.

Comme résines siloxysilicates, on peut citer les résines triméthylsiloxysilicates commercialisées sous la référence SR1000, par la société GENERAL ELECTRIC, ou sous la référence TMS803 par la société WACKER. On peut également citer les résines triméthylsiloxysilicates commercialisées sous la dénomination KF-7312J par la société SHIN-ETSU, et les résines DC749, DC593 commercialisées par la société DOW CORNING.

Comme polymères siliconés liposolubles, on peut également mentionner les polyamides siliconés du type polyorganosiloxane, tels que ceux décrits dans les documents US 5 874 069, US 5 919 441, US 6 051 216 et US 5 981 680.

Selon un mode de réalisation de l'invention, le polymère filmogène peut être choisi parmi les polymères ayant un squelette organique non siliconé greffé par des monomères contenant un polysiloxane. Ces polymères peuvent être liposolubles, lipodispersibles, hydrosolubles ou dispersibles en milieu aqueux, le cas échéant.

Les polymères ayant un squelette organique non siliconé greffé par des monomères contenant un polysiloxane sont constitués d'une chaîne organique principale formée de monomères organiques ne comprenant pas la silicone, sur laquelle on greffe, au sein de ladite chaîne ainsi qu'éventuellement sur au moins l'une des extrémités de celle-ci, au moins un macromère de polysiloxane.

Dans ce qui suit, on doit comprendre que l'expression « macromère de polysiloxane » désigne, ainsi qu'il est généralement accepté, tout monomère contenant une chaîne polymère du type polysiloxane dans sa structure.

Les monomères organiques non siliconés constituant la chaîne principale du polymère siliconé greffé peuvent être choisis parmi les monomères à insaturation éthylénique pouvant être polymérisés par la méthode radicalaire, les monomères polymérisables par polycondensation tels que ceux formant les polyamides, les polyesters, les polyuréthanes, les monomères à cycle ouvrant tels que ceux du type oxazoline ou caprolactone.

Les polymères ayant un squelette organique non siliconé greffé par des monomères contenant un polysiloxane selon la présente invention peuvent être obtenus conformément à toute méthode connue de l'homme de l'art, en particulier par la réaction entre (i) un macromère de polysiloxane de départ correctement fonctionnalisé sur la chaîne de polysiloxane et (ii) un ou plusieurs composés organiques non siliconés, eux même correctement fonctionnalisés par une fonction qui est capable de réagir avec le groupement ou les groupements fonctionnel(s) porté(s) par ladite silicone en formant une

liaison covalente ; un exemple classique d'une telle réaction est la réaction radicalaire entre un groupement vinyle porté à l'une des extrémités de la silicone avec une double liaison d'un monomère à insaturation éthylénique de la chaîne principale.

Les polymères ayant un squelette organique non siliconé greffé par des monomères contenant un polysiloxane selon l'invention sont choisis de préférence parmi ceux décrits dans les brevets US 4 693 935, US 4 728 571 et US 4 972 037 et les demandes de brevet EP-A-0 412 704, EP-A-0 412 707, EP-A-0 640 105 et WO 95/00578. Il concerne des copolymères obtenus par la polymérisation radicalaire à partir de monomères à insaturation éthylénique et de monomères ayant un groupement terminal vinyle, ou bien des copolymères obtenus par la réaction d'une polyoléfine contenant des groupements fonctionnalisés et un macromère de polysiloxane ayant une fonction terminale réactive avec lesdits groupements fonctionnalisés.

Une famille particulière de polymères siliconés greffés convenables pour la mise en oeuvre de la présente invention est constituée par les polymères siliconés greffés contenant :

a) de 0 à 98% en poids d'au moins un monomère lipophile (A) de faible polarité lipophile à insaturation éthylénique, polymérisable par la méthode radicalaire ;
b) de 0 à 98% en poids d'au moins un monomère polaire hydrophile (B) à insaturation éthylénique, copolymérisable avec le monomère ou les monomères du type (A) ;
c) de 0,01 à 50% en poids d'au moins un macromère de polysiloxane (C) de formule générale (XXVII) :

$$X(Y)_nSi(R)_{3-m}Z_m \qquad (XXVII)$$

dans laquelle :

- X désigne un groupement vinyle copolymérisable avec les monomères (A) et (B) ;
- Y désigne un groupement ayant une liaison divalente ;
- R désigne hydrogène, alkyle ou alkoxy en $C_1$-$C_6$, aryle en $C_6$-$C_{12}$ ;
- Z désigne un motif polysiloxane monovalent ayant un poids moléculaire moyen en nombre d'au moins 500 ;
- n vaut 0 ou 1 et m est un nombre entier allant de 1 à 3 ; les pourcentages étant calculés par rapport au poids total des monomères (A), (B) et (C).

Ces polymères ont un poids moléculaire moyen en nombre allant de 10 000 à 2 000 000, et préférablement une température de transition vitreuse Tg ou une température de fusion cristalline Tm d'au moins -20°C.

Comme exemples de monomères lipophiles (A), on peut citer les esters d'alcool en $C_1$-$C_{18}$ et de l'acide acrylique ou méthacrylique ; les esters d'alcool en $C_{12}$-$C_{30}$ et de l'acide méthacrylique, le styrène ; les macromères de polystyrène ; l'acétate de vinyle ; le propionate de vinyle ; l'alpha-méthylstyrène ; le tertio-butylstyrène ; le butadiène ; le cyclohexadiène; l'éthylène ; le propylène ; le vinyltoluène, les esters de l'acide acrylique ou méthacrylique et de 1,1-dihydroperfluoroalcanols ou d'homologues de ceux-ci ; les esters de l'acide acrylique ou méthacrylique et d'omega-hydrofluoroalcanols ; les esters de l'acide acrylique ou méthacrylique et de fluoroalkylsulfonamidoalcools ; les esters de l'acide acrylique ou méthacrylique et de fluoroalkylalcools ; les esters de l'acide acrylique ou méthacrylique et d'alcoolfluoroéthers ; ou des mélanges de ceux-ci. Les monomères (A) préférés sont choisis au sein du groupe constitué par le méthacrylate de n-butyle, le méthacrylate d'isobutyle, l'acrylate de tertio-butyle, le méthacrylate de tertio-butyle, le méthacrylate de 2-éthylhexyle, le méthacrylate de méthyle, l'acrylate de 2-(N-méthylperfluorooctanesulfonamido)éthyle, l'acrylate de 2-(N-butylperfluorooctanesulfona-mido)éthyle, ou des mélanges de ceux-ci.

Comme exemples de monomères (B) polaires, on peut citer l'acide acrylique, l'acide méthacrylique, le N, N-diméthylacrylamide, le méthacrylate de diméthylaminoéthyle, le méthacrylate de diméthylaminoéthyle quaternisé, le (méth)acrylamide, le N-t-butylacrylamide, l'acide maléique, l'anhydride maléique et les hemi-esters de ceux-ci, les (méth) acrylates d'hydroxyalkyle, le chlorure de diallyldiméthylammonium, la vinylpyrrolidone, les éthers vinyliques, les maléimides, la vinylpyridine, le vinylimidazole, les composés polaires vinyliques et hétérocycliques, le sulfonate de styrène, l'alcool allylique, l'alcool vinylique, le vinylcaprolactame ou des mélanges de ceux-ci. Les monomères (B) sont choisis de préférence au sein du groupe constitué par l'acide acrylique, le N,N-diméthylacrylamide, le méthacrylate de diméthylaminoéthyle, le méthacrylate de diméthylaminoéthyle quaternisé, la vinylpyrrolidone, et des mélanges de ceux-ci.

On cite notamment le produit KP 561 ou le KP 562 commercialisé par SHIN ETSU tel que le monomère (A) et choisi parmi les esters d'alcool en $C_{18}$ à $C_{22}$ et de l'acide méthacrylique.

## MATIERES COLORANTES

La composition cosmétique conforme à l'invention peut, avantageusement, incorporer un ou plusieurs agents de coloration, au moins une matière colorante, organique ou inorganique, notamment de type pigments ou nacres

classiquement utilisée dans les compositions cosmétiques.

Par « pigments », il faut comprendre des particules blanches ou colorées, minérales ou organiques, insolubles dans une solution aqueuse, destinées à colorer et/ou opacifier le film résultant.

Les pigments peuvent être présents à raison de 0,01 à 15 % en poids, notamment de 0,01 à 10 % en poids, et en particulier de 0,02 à 5 % en poids, par rapport au poids total de la composition cosmétique. Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome.

Il peut également s'agir de pigment ayant une structure qui peut être par exemple de type séricite/oxyde de fer brun/dioxyde de titane/silice. Un tel pigment est commercialisé par exemple sous la référence COVERLEAF NS ou JS par la société CHEMICALS AND CATALYSTS et présente un rapport de contraste voisin de 30.

La matière colorante peut encore comporter un pigment ayant une structure qui peut être par exemple de type microsphères de silice contenant de l'oxyde de fer. Un exemple de pigment présentant cette structure est celui commercialisé par la société MIYOSHI sous la référence PC BALL PC-LL-100 P, ce pigment étant constitué de microsphères de silice contenant de l'oxyde de fer jaune.

Parmi les pigments organiques utilisables dans l'invention, on peut citer le noir de carbone, les pigments de type D & C, les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537.

Par « nacres », il faut comprendre des particules colorées de toute forme, irisées ou non, notamment produites par certains mollusques dans leur coquille ou bien synthétisées et qui présentent un effet de couleur par interférence optique.

Les nacres peuvent être choisies parmi les pigments nacrés tels que le mica titane recouvert avec un oxyde de fer, le mica titane recouvert d'oxychlorure de bismuth, le mica titane recouvert avec de l'oxyde de chrome, le mica titane recouvert avec un colorant organique ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

Parmi les nacres disponibles sur le marché, on peut citer les nacres TIMICA, FLAMENCO et DUOCHROME (sur base de mica) commercialisées par la société ENGELHARD, les nacres TIMIRON commercialisées par la société MERCK, les nacres sur base de mica PRESTIGE commercialisées par la société ECKART et les nacres sur base de mica synthétique SUNSHINE commercialisées par la société SUN CHEMICAL.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres roses notamment commercialisées par la société ENGELHARD sous la dénomination Tan opale G005 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

La composition cosmétique selon l'invention peut comprendre également des colorants hydrosolubles ou liposolubles en une teneur allant de 0,01 à 10 % en poids, notamment allant de 0,01 à 5 % en poids par rapport au poids total de la composition cosmétique. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine Les colorants hydrosolubles sont par exemple le jus de betterave, le bleu de méthylène.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques comme par exemple les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :

- les particules d'au moins un métal et/ou d'au moins un dérivé métallique,
- les particules comportant un substrat, organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple les bronzes et les laitons) sont des métaux préférés.

Par « dérivés métalliques », on désigne des composés dérivés de métaux notamment des oxydes, des fluorures, des chlorures et des sulfures

A titre illustratif de ces particules, on peut citer des particules d'aluminium, telles que celles commercialisées sous les dénominations STARBRITE 1200 EAC® par la société SIBERLINE et METALURE® par la société ECKART.

On peut également citer les poudres métalliques de cuivre ou des mélanges d'alliage telles les références 2844 commercialisées par la société RADIUM BRONZE, les pigments métalliques comme l'aluminium ou le bronze, telles que celles commercialisées sous les dénominations ROTOSAFE 700 de la société ECKART, les particules d'aluminium enrobé de silice commercialisées sous la dénomination VISIONAIRE BRIGHT SILVER de la société ECKART et les particules d'alliage métallique comme des poudres de bronze (alliage cuivre et zinc) enrobé de silice commercialisées sous la dénomination de Visionaire Bright Natural Gold de la société Eckart.

Il peut s'agir de particules comportant un substrat de verre comme celles commercialisées par la société NIPPON SHEET GLASS sous les dénominations MICROGLASS METASHINE.

L'agent de coloration goniochromatique peut être choisi par exemple parmi les structures multicouches interférentielles et les agents de coloration à cristaux liquides.

Des exemples de structures multicouche interférentielles symétriques utilisables dans des compositions réalisées conformément à l'invention sont par exemple les structures suivantes : $Al/SiO_2/Al/SiO_2/Al$, des pigments ayant cette structure étant commercialisés par la société DUPONT DE NEMOURS ; $Cr/MgF_2/Al/MgF_2/Cr$, des pigments ayant cette structure étant commercialisés sous la dénomination CHROMAFLAIR par la société FLEX ; $MoS_2/SiO_2/Al/SiO_2/MoS_2$ ; $Fe_2O_3/SiO_2/Al/SiO_2/Fe_2O_3$, et $Fe_2O_3/SiO_2/Fe_2O_3/SiO_2/Fe_2O_3$, des pigments ayant ces structures étant commercialisés sous la dénomination SICOPEARL par la société BASF ; $MoS_2/SiO_2$/mica-oxyde/$SiO_2/MoS_2$ ; $Fe_2O_3/SiO_2$/mica-oxyde/$SiO_2/Fe_2O_3$ ; $TiO_2/SiO_2/TiO_2$ et $TiO_2/Al_2O_3/TiO_2$ ; $SnO/TiO_2/SiO_2/TiO_2/SnO$; $Fe_2O_3/SiO_2/Fe_2O_3$ ; SnO/mica/$TiO_2/SiO_2/TiO_2$/mica/SnO, des pigments ayant ces structures étant commercialisés sous la dénomination XIRONA par la société MERCK (Darmstadt). A titre d'exemple, ces pigments peuvent être les pigments de structure silice/oxyde de titane/oxyde d'étain commercialisés sous le nom XIRONA MAGIC par la société MERCK, les pigments de structure silice/oxyde de fer brun commercialisés sous le nom XIRONA INDIAN SUMMER par la société MERCK et les pigments de structure silice/oxyde de titane/mica/oxyde d'étain commercialisés sous le nom XIRONA CARRIBEAN BLUE par la société MERCK. On peut encore citer les pigments INFINITE COLORS de la société SHISEIDO. Selon l'épaisseur et la nature des différentes couches, on obtient différents effets. Ainsi, avec la structure $Fe_2O_3/SiO_2/Al/ SiO_2/Fe_2O_3$ on passe du doré-vert au gris-rouge pour des couches de $SiO_2$ de 320 à 350 nm ; du rouge au doré pour des couches de $SiO_2$ de 380 à 400 nm ; du violet au vert pour des couches de $SiO_2$ de 410 à 420 nm ; du cuivre au rouge pour des couches de $SiO_2$ de 430 à 440 nm.

On peut citer, à titre d'exemple de pigments à structure multicouche polymérique, ceux commercialisés par la société 3M sous la dénomination COLOR GLITTER.

Comme particules goniochromatiques à cristaux liquides, on peut utiliser par exemple celles vendues par la société CHENIX ainsi que celle commercialisées sous la dénomination HELICONE® HC par la société WACKER.

## CHARGES

De manière avantageuse, les compositions cosmétiques conformes à l'invention peuvent également comprendre au moins une charge, de nature organique ou minérale, permettant notamment de leur conférer une stabilité améliorée au regard de l'exsudation.

Par « charge », il faut comprendre les particules incolores ou blanches, solides de toutes formes, qui se présentent sous une forme insoluble et dispersée dans le milieu de la composition. De nature minérale ou organique, elles permettent de conférer du corps ou de la rigidité à la composition, et/ou de la douceur, de la matité et de l'uniformité au maquillage.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, en particulier elles peuvent être traitées en surface par des silicones, des acides aminés, des dérivés fluorés ou toute autre substance favorisant la dispersion et la compatibilité de la charge dans la composition.

Au sens de la présente invention, les termes « charges minérales » et « charges inorganiques » sont utilisés de manière interchangeable.

Parmi les charges minérales utilisables dans les compositions selon l'invention, on peut citer le talc, le mica, la silice, le siloxysilicate de triméthyle, le kaolin, la bentone, le carbonate de calcium précipité, le carbonate et l'hydrogéno-carbonate de magnésium, l'hydroxyapatite, le nitrure de bore, les microsphères de silice creuses (SILICE BEADS de MAPRECOS), les microcapsules de verre ou de céramique, les charges à base de silice comme l'AEROSIL 200, l'AEROSIL 300 ; le SUNSPHARE L-31, le SUNPHARE H-31 commercialisés par ASAHI GLASS ; le CHEMICELEN commercialisé par ASAHI CHEMICAL ; les composites de silice et de dioxyde de titane comme la série TSG commercialisée par NIPPON SHEET GLASS, et leurs mélanges.

Parmi les charges organiques utilisables dans les compositions selon l'invention on peut citer les poudres de polyamide (NYLON® ORGASOL de chez ATOCHEM), de poly-b-alanine et polyéthylène, les poudres de polytétrafluoroéthylène (TEFLON® ), la lauroy-lysine, l'amidon, les poudres de polymères de tétrafluoroéthylène, les microsphères creuses de polymères telles l'EXPANCEL (NOBEL INDUSTRIE), le carbonate de calcium précipité, le carbonate et l'hydrogéno-carbonate de magnésium, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, le POLYPORE® L 200 (CHEMDAL CORPORATION), les microbilles de résine de silicone (TOSPEARL® de TOSHIBA, par exemple), les poudres de poyuréthanne, en particulier les poudres de polyuréthanne réticulé comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI, et leurs mélanges.

Les charges peuvent être présentes dans les compositions cosmétiques conformes à l'invention à raison de 0,001 à 35 % du poids total de la composition, de préférence 0,5 à 15 %.

La charge peut être par exemple une charge dont la granulométrie moyenne est inférieure à 100 $\mu$m, notamment comprise entre 1 et 50 $\mu$m, par exemple entre 4 et 20 $\mu$m.

Selon un mode de réalisation particulier, la composition selon l'invention comprend au moins une charge qui est présente à raison de 0,01 % à 60 % du poids total de la composition, en particulier de 0,5 à 20 %, plus particulièrement de 1 à 10 % en poids par rapport au poids total de la composition.

## ADDITIFS

Les compositions cosmétiques selon l'invention peuvent également comprendre tout additif usuellement utilisé dans le domaine concerné, choisi parmi des gélifiants tels que décrits dans la demande WO 2004/55080, des agents tensioactifs tels que décrits dans la demande FR 2 834 452, des gommes, des antioxydants, des huiles essentielles, des conservateurs, des parfums, des neutralisants, des agents hydratants, des agents antiseptiques, des vitamines comme la vitamine B3 ou la vitamine E et leurs dérivés, et des agents protecteurs contre les UV.

Selon un mode particulier de réalisation, les tensioactifs siliconés convenant avantageusement à la mise en oeuvre des compositions conformes à la présente invention sont de type non réticulé.

Selon un autre mode particulier de réalisation, les compositions conformes à la présente invention sont avantageusement dénuées de tensioactifs appartenant à la famille des tensioactifs de type ammonium.

Bien entendu, l'homme de l'art veillera à choisir le ou les éventuel(s) additif(s) ajoutés à la composition cosmétique selon l'invention, de manière telle que les propriétés avantageuses attachées intrinsèquement à la composition conforme à l'invention ne soit pas, ou substantiellement pas, altérées par l'addition envisagée.

Selon une variante particulière, le polymère de silicone de formule générale (I) utilisé dans les compositions

cosmétiques conformes à la présente invention, est choisi parmi la diméthicone de polyglycéryle-3 polydiméthylsiloxyéthyle, la diméthicone de lauryle polyglycéryle-3 polydiméthylsiloxyéthyle, la diméthicone de polyglycéryle-3 disiloxane, et leurs mélanges.

Selon une autre variante, le polymère de silicone de formule générale (I) utilisé dans les compositions cosmétiques conformes à la présente invention, est choisi parmi les polymères de silicone commercialisés par la société SHIN-ETSU sous les références KF 6100® , KF 6104® , KF 6105® , et leurs mélanges.

Selon une autre variante, la composition cosmétique selon l'invention combine la diméthicone de polyglycéryle-3 polydiméthylsiloxyéthyle et, en tant qu'agent filmogène, le copolymère d'acrylate/acrylate de stéaryle/méthacrylate de diméthicone, notamment celui commercialisé sous la référence KP 561® par la société SHIN-ETSU.

Selon encore une autre variante, la composition cosmétique selon l'invention combine la diméthicone de polyglycéryle-3 polydiméthylsiloxyéthyle et au moins une cire, notamment choisie parmi les cires de polyéthylène, la cire de Candelilla, la cire de cocoglycéride hydrogénée, et leurs mélanges.

Il apparaît de manière évidente que certains composants utilisables dans les compositions cosmétiques selon la présente invention, peuvent appartenir simultanément à différentes classes de composés. Ainsi, il ne sort pas du cadre de travail habituel de l'homme du métier d'ajuster la quantité d'un composé appartenant à différentes classes, de manière à ce que sa présence dans la formulation se traduise par l'effet désiré, et corresponde, le cas échéant, à l'effet susceptible d'être obtenu par la présence de composés appartenant auxdites différentes classes.

La composition cosmétique selon l'invention peut notamment se présenter sous la forme d'un produit de maquillage des lèvres, en particulier un rouge à lèvres, ou un baume à lèvres.

Les exemples de compositions ci-après sont donnés à titre illustratif et sans caractère limitatif de l'invention.

**Exemple 1 : Rouge à lèvres**

On prépare un rouge à lèvres comprenant la diméthicone de polyglycéryl-3 polydiméthylsiloxyéthyle commercialisée sous la référence KF6104® par SHIN-ETSU, et dont la composition est précisée en tableau I.

Tableau I

| | Pourcentages massiques |
|---|---|
| Polyisobutène hydrogéné (Parleam HV de NOF) | 4 |
| Isononanoate d'isononyle | 12 |
| 2-Octyldodécanol | 4,5 |
| Malate de diisostéaryle | 33,9 |
| Triglycérides d'acides lauriques/palmitique/cétylique/stéarique 50/20/10/10 (Softisan 100 ®de Sasol) | 4 |
| N-lauroyl L-lysine | 1 |
| Copolymère d'acrylates/acrylate de stéaryle/méthacrylate de diméthicone (KP 561 P ® de Shin Etsu) | 4 |
| Diméthicone 6 cSt (KF 96) | 4 |
| Diméthicone de polyglycéryl-3 polydiméthylsiloxyéthyle (KF6104 ® de Shin Etsu) | 13 |
| Conservateur | qs |
| Cire de Polyéthylène (PM 500) | 6,6 |
| Cire de polyéthylène (PM 400) | 3,8 |
| Silice pyrogénée hydrophobe traitée en surface par diméthylsilane (Aerosil R 972 ® de Degussa) | 2 |
| Pigments | 7,0 |
| Siméthicone (Antifoam C ®de Dow Corning) | 0,2 |
| Total | 100 |

*Mode opératoire*

Une phase huileuse est préparée en mélangeant à chaud (environ 95 °C) l'isononanoate d'isononyle, le 2-octyldodécanol, le malate de diisostéaryle, avec la diméthicone de polyglycéryl-3 polydiméthylsiloxyéthyle et l'huile dimethicone. La phase huileuse ainsi préparée est maintenue sous agitation à environ 95 °C, et les charges (N-lauroyl L-lysine et silice pyrogénée) sont ajoutées au mélange.

Puis sont rajoutés au mélange les cires, les pigments sous la forme d'une pâte pigmentaire, le polyisobutène hydrogénée et la siméthicone.

Le mélange ainsi obtenu est ensuite coulé dans un moule de rouge à lèvres et laissé refroidir jusqu'à l'obtention d'une composition solide.

Puis on mesure la tenue, le confort et la brillance de cette composition selon les protocoles décrits plus haut dans la description.

On mesure également, selon les mêmes méthodes, la tenue et la brillance de deux produits commerciaux :

- Témoin A : Jelly Plumpy commercialisé par Maybelline contenant de la phényltriméthicone à titre de polymère de silicone,

- Témoin B : Aube Rouge Glacé commercialisé par KAO et contenant la silicone glycérolé de formule suivante, à titre de polymère de silicone

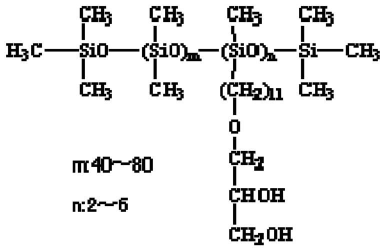

Les résultats obtenus sont reportés dans le tableau II ci-dessous.

Tableau II

| Formule | Tenue à l'eau | Tenue à l'huile | Transfert | Indice de confort | Brillance moyenne |
|---|---|---|---|---|---|
| Témoin A | 14,4 | 23,4 | 47,3 | | 42,5 |
| Témoin B | 7,0 | 8,5 | 42,0 | 85,6 | 48,2 |
| Invention | 5,9 | 7,9 | 32,1 | 99,5 | 48,3 |

Dans ces mesures, la couleur de la face interne de l'avant-bras est telle que L* = 63,9, a* = 8,4, b* = 13,3, et la couleur du mouchoir en papier est L* = 97,9, a* = 0,6 et b* = 3,3.

Le rouge à lèvres conforme à l'invention présente une meilleure tenue et un meilleur confort que les produits commerciaux A et B de l'art antérieur , pour une brillance équivalente voir égale.

En outre, le rouge à lèvres conforme à l'invention migre trois à quatre fois moins que le témoin B de l'art antérieur.

**Exemples 2 et 3: Rouge à lèvres**

Les pourcentages sont en poids

| | Exemple 2 | Exemple 3 |
|---|---|---|
| Polyisobutène hydrogéné (Parleam HV de NOF) | | 4 |
| Polyisobutène hydrogéné (Parleam Lite de NOF) | 6 | |
| Isononanoate d'isononyle | 8 | 12 |
| 2-Octyldodécanol | 4,5 | 4,5 |
| Malate de diisostéaryle | 33,9 | 23,9 |
| Polyglycéryl-2-diisostéarate (Cosmol 42 V de Nishin Oil) | | 5 |
| Polyglycéryl-2 triisostéarate (Cosmol 43 N de Nishin Oil) | | 4 |
| Sorbitan Sesquiloléate (Cosmol 82 de Nishin Oil) | | 1 |
| Triglycérides d'acides lauriques/palmitique/cétylique/stéarique 50/20/10/10 (Softisan 100 ®de Sasol) | 3 | 4 |
| N-lauroyl L-lysine | 1 | 1 |
| Polylaurate de vinyle | 3 | |
| Copolymère d'acrylates/acrylate de stéaryle/méthacrylate de diméthicone (KP 561 P ® de Shin Etsu) | 2 | 4 |
| Diméthicone 6 cSt (KF 96) | 5 | 4 |
| Diméthicone de polyglycéryl-3 polydiméthylsiloxyéthyle (KF6104 ® de Shin Etsu) | 13 | 13 |
| Conservateur | qs | |
| Cire de Polyéthylène (PM 500) | 5,5 | 6,6 |
| Cire de polyéthylène (PM 400) | 1,1 | |
| Cire de Candellila | 4,8 | |
| Cire microcristalline | | 3,8 |
| Silice pyrogénée hydrophobe traitée en surface par diméthylsilane (Aerosil R 972 ® de Degussa) | 2 | 2 |

| | | |
|---|---|---|
| Pigments | 7,0 | 7 |
| Siméthicone (Antifoam C ®de Dow Corning) | 0,2 | 0,2 |
| Total | 100 | 100 |

## Revendications

**1.** Composition cosmétique anhydre, **caractérisée en ce qu'**elle comprend, dans un milieu physiologiquement acceptable, au moins un polymère de silicone de formule générale (I) :

$$R^1_a\ R^2_b\ R^3_c\ SiO_{(4-a-b-c)/2} \qquad (I)$$

dans laquelle :

- a, b et c sont tels que a varie de 1 à 2,5 ; b et c, indépendamment l'un de l'autre, varient de 0,001 à 1,5 ;
- $R^1$, identique ou différent, est choisi parmi :
  - les radicaux alkyle en $C_1$ à $C_{30}$, le cas échéant substitués par un ou plusieurs atomes de fluor, groupements amino et/ou carboxy,
  - les radicaux aryle, aralkyle,
  - les radicaux de formule générale (II) :

$$-C_dH_{2d}-O-(C_2H_4O)_e(C_3H_6O)_fR^4 \qquad (II)$$

avec :

- $R^4$ étant un radical hydrocarboné en $C_1$ à $C_{30}$ ou un radical $R^5$-(CO)- avec $R^5$ étant un radical hydrocarboné en $C_1$ à $C_{30}$, et
- d, e et f étant des entiers tels que d varie de 0 à 15, e et f, indépendamment l'un de l'autre, varient de 0 à 50, et

- leurs combinaisons,

- $R^2$ est un radical représenté par la formule générale (III) :

$$-Q-O-X \qquad (III)$$

avec :

- Q étant un radical hydrocarboné bivalent en $C_2$ à $C_{20}$ pouvant inclure au moins une liaison éther et/ou au moins une liaison ester, et
- X étant un radical hydrocarboné polyhydroxylé,

sous réserve que lorsque $R^2$ est un groupement de formule générale (IIIA) :

$$-C_3H_6-O[CH_2CH(OH)CH_2O]_nH \qquad (IIIA)$$

dans laquelle n est un entier variant de 1 à 5, alors $R^1$ est différent d'un radical alkyle en $C_{12}$,

- $R^3$ est un groupement organosiloxane de formule générale (IV) :

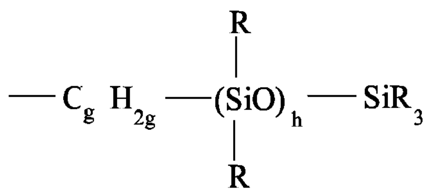 (IV)

avec :

- chacun des radicaux R représentant, indépendamment l'un de l'autre, un radical choisi parmi les radicaux alkyle en $C_1$ à $C_{30}$, le cas échéant substitués par un ou plusieurs atomes de fluor ; et les radicaux aryle et aralkyle,
- g et h étant des entiers tels que g varie de 1 à 5 et h varie de 0 à 500.

2. Composition cosmétique anhydre, **caractérisée en ce qu'**elle comprend, dans un milieu physiologiquement acceptable, au moins un polymère de silicone de formule générale (I) :

$$R^1_a R^2_b R^3_c SiO_{(4-a-b-c)/2} \quad (I)$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :

  - les radicaux alkyle en $C_1$ à $C_{30}$, le cas échéant substitués par un ou plusieurs atomes de fluor, groupements amino et/ou carboxy,
  - les radicaux aryle, aralkyle,
  - les radicaux de formule générale (II) :

$$-C_dH_{2d}-O-(C_2H_4O)_e(C_3H_6O)_fR^4 \quad (II)$$

  avec :

    - $R^4$ étant un radical hydrocarboné en $C_1$ à $C_{30}$ ou un radical $R^5$-(CO)- avec $R^5$ étant un radical hydrocarboné en $C_1$ à $C_{30}$, et
    - d, e et f étant des entiers tels que d varie de 0 à 15, e et f, indépendamment l'un de l'autre, varient de 0 à 50, et

  - leurs combinaisons,

- $R^2$ est un radical représenté par la formule générale (III) :

$$-Q-O-X \quad (III)$$

avec :

  - Q étant un radical hydrocarboné bivalent en $C_2$ à $C_{20}$ pouvant inclure au moins une liaison éther et/ou au moins une liaison ester et
  - X étant un radical hydrocarboné polyhydroxylé,

- $R^3$ est un organosiloxane de formule générale (IV) :

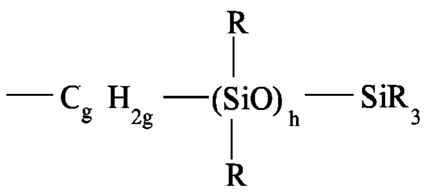

(IV)

avec :

- chacun des radicaux R représentant, indépendamment l'un de l'autre, un radical choisi parmi les radicaux alkyle en $C_1$ à $C_{30}$, le cas échéant substitué par un ou plusieurs atomes de fluor ; et les radicaux aryle, aralkyle,
- g et h étant des entiers tels que g varie de 1 à 5 et h varie de 0 à 500,

et au moins une huile de silicone.

**3.** Composition cosmétique anhydre, **caractérisée en ce qu'**elle comprend, dans un milieu physiologiquement acceptable, au moins un polymère de silicone de formule générale (I) :

$$R^1_a R^2_b R^3_c SiO_{(4-a-b-c)/2} \qquad (I)$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :
  - les radicaux alkyle en $C_1$ à $C_{30}$, le cas échéant substitués par un ou plusieurs atomes de fluor, groupements amino et/ou carboxylique,
  - les radicaux aryle, aralkyle,
  - les radicaux de formule générale (II) :

$$—C_dH_{2d}\text{-O-}(C_2H_4O)_e(C_3H_6O)_fR^4 \qquad (II)$$

  avec :

    - $R^4$ étant un radical hydrocarboné en $C_1$ à $C_{30}$ ou un radical $R^5$-(CO)- avec $R^5$ étant un radical hydrocarboné en $C_1$ à $C_{30}$, et
    - d, e et f étant des entiers tels que d varie de 0 à 15, e et f, indépendamment, l'un de l'autre varient de 0 à 50,

  - leurs combinaisons,

- $R^2$ est un radical de formule générale (III) :

$$\text{-Q-O-X} \qquad (III)$$

avec :

  - Q étant un radical hydrocarboné bivalent en $C_2$ à $C_{20}$ pouvant inclure au moins une liaison éther et/ou au moins une liaison ester,
  - X étant un radical hydrocarboné polyhydroxylé, et

- $R^3$ est un organosiloxane de formule générale (IV) :

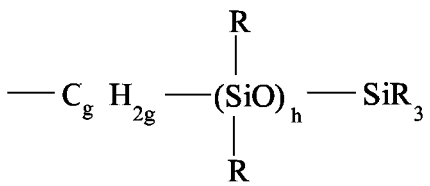

(IV)

avec :

- chacun des radicaux R représentant, indépendamment l'un de l'autre, un radical choisi parmi les radicaux alkyle en $C_1$ à $C_{30}$, le cas échéant substitué par un ou plusieurs atomes de fluor ;et les radicaux aryle, aralkyle,
- g et h étant des entiers tels que g varie de 1 à 5 et h varie de 0 à 500,

ledit polymère étant tel que, lorsqu'il est en quantité suffisante dans la composition, la valeur de transfert d'un dépôt de ladite composition, une fois étalée sur un support, est inférieure ou égale à 45.

**4.** Composition cosmétique anhydre, **caractérisée en ce qu'**elle comprend, dans un milieu physiologiquement acceptable, au moins un polymère de silicone de formule générale (I) :

$$R^1_a R^2_b R^3_c SiO_{(4-a-b-c)/2} \qquad (I)$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :

  - les radicaux alkyle en $C_1$ à $C_{30}$, le cas échéant substitués par un ou plusieurs atomes de fluor, groupements amino et/ou carboxylique,
  - les radicaux aryle, aralkyle,
  - les radicaux de formule générale (II) :

$$— C_dH_{2d}\text{-}O\text{-}(C_2H_4O)_e(C_3H_6O)_fR^4 \qquad (II)$$

  avec :

    - $R^4$ étant un radical hydrocarboné en $C_1$ à $C_{30}$ ou un radical $R^5$-(CO)- avec $R^5$ étant un radical hydrocarboné en $C_1$ à $C_{30}$, et
    - d, e et f étant des entiers tels que d varie de 0 à 15, e et f, indépendamment, l'un de l'autre varient de 0 à 50, et

  - leurs combinaisons

- $R^2$ est un radical de formule générale (III) :

$$\text{-Q-O-X} \qquad (III)$$

avec :

  - Q étant un radical hydrocarboné bivalent en $C_2$ à $C_{20}$ pouvant inclure au moins une liaison éther et/ou au moins une liaison ester,
  - X étant un radical hydrocarboné polyhydroxylé, et

- $R^3$ est un organosiloxane de formule générale (IV) :

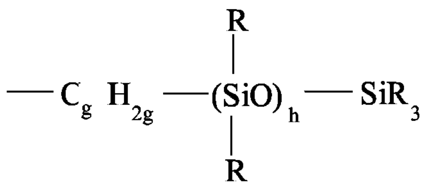 (IV)

avec :

- chacun des radicaux R représentant, indépendamment l'un de l'autre, un radical choisi parmi les radicaux alkyle en $C_1$ à $C_{30}$, le cas échéant substitué par un ou plusieurs atomes de fluor ; et les radicaux aryle, aralkyle,
- g et h étant des entiers tels que g varie de 1 à 5 et h varie de 0 à 500,

ledit polymère étant tel que, lorsqu'il est en quantité suffisante dans la composition, la tenue de la couleur à l'eau d'un dépôt de ladite composition, une fois étalée sur un support, est inférieure ou égale à 15.

**5.** Composition cosmétique anhydre, **caractérisée en ce qu'**elle comprend, dans un milieu physiologiquement acceptable, au moins un polymère de silicone de formule générale (I) :

$$R^1_a\ R^2_b\ R^3_c\ SiO_{(4-a-b-c)/2} \qquad (I)$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :

  - les radicaux alkyle en $C_1$ à $C_{30}$, le cas échéant substitués par un ou plusieurs atomes de fluor, groupements amino et/ou carboxylique,
  - les radicaux aryle, aralkyle,
  - les radicaux de formule générale (II) :

$$— C_dH_{2d}\text{-O-}(C_2H_4O)_e(C_3H_6O)_fR^4 \qquad (II)$$

  avec :

    - $R^4$ étant un radical hydrocarboné en $C_1$ à $C_{30}$ ou un radical $R^5$-(CO)- avec $R^5$ étant un radical hydrocarboné en $C_1$ à $C_{30}$, et
    - d, e et f étant des entiers tels que d varie de 0 à 15, e et f, indépendamment, l'un de l'autre varient de 0 à 50, et

  - leurs mélanges

- $R^2$ est un radical de formule générale (III) :

$$\text{-Q-O-X} \qquad (III)$$

avec :

  - Q étant un radical hydrocarboné bivalent en $C_2$ à $C_{20}$ pouvant inclure au moins une liaison éther et/ou au moins une liaison ester,
  - X étant un radical hydrocarboné polyhydroxylé, et

- $R^3$ est un organosiloxane de formule générale (IV) :

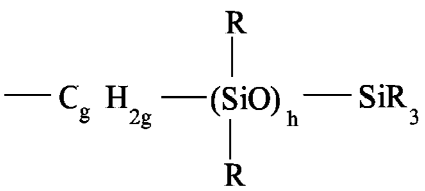

(IV)

avec :

- chacun des radicaux R représentant, indépendamment l'un de l'autre, un radical choisi parmi les radicaux alkyle en $C_1$ à $C_{30}$, le cas échéant substitué par un ou plusieurs atomes de fluor ; et les radicaux aryle, aralkyle,
- g et h étant des entiers tels que g varie de 1 à 5 et h varie de 0 à 500,

ledit polymère étant tel que, lorsqu'il est en quantité suffisante dans la composition, la tenue de la couleur à l'huile d'un dépôt de ladite composition, une fois étalée sur un support, est inférieure ou égale à 25.

**6.** Composition cosmétique anhydre, **caractérisée en ce qu'**elle comprend, dans un milieu physiologiquement acceptable, au moins un polymère de silicone de formule générale (I) :

$$R^1_a\ R^2_b\ R^3_c\ SiO_{(4-a-b-c)/2} \qquad (I)$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :

  - les radicaux alkyle en $C_1$ à $C_{30}$, le cas échéant substitués par un ou plusieurs atomes de fluor, groupements amino et/ou carboxylique,
  - les radicaux aryle, aralkyle,
  - les radicaux de formule générale (II) :

$$— C_dH_{2d}\text{-}O\text{-}(C_2H_4O)_e(C_3H_6O)_fR^4 \qquad (II)$$

  avec :

    - $R^4$ étant un radical hydrocarboné en $C_1$ à $C_{30}$ ou $R^5$-(CO)-avec $R^5$ étant un radical en $C_1$ à $C_{30}$,
    - d, e et f étant des entiers tels que d varie de 0 à 15, e et f, indépendamment l'un de l'autre, varient de 0 à 50,

  - et leurs combinaisons,

- $R^2$ est un radical de formule générale (III) :

$$\text{-Q-O-X} \qquad (III)$$

avec :

  - Q étant un radical hydrocarboné bivalent de $C_2$ à $C_{20}$ pouvant inclure au moins une liaison éther et/ou au moins une liaison ester, et
  - X étant un radical hydrocarboné polyhydroxylé,

- $R^3$ est un organosiloxane de formule générale (IV) :

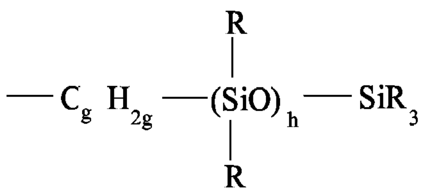

(IV)

avec :

- chacun des groupes R représentant, indépendamment l'un de l'autre, un radical choisi parmi les radicaux alkyle en $C_1$ à $C_{30}$, le cas échéant substitué par un ou plusieurs atomes de fluor, ;les radicaux aryle, aralkyle,
- g et h étant des entiers tels que g varie de 1 à 5 et h varie de 0 à 500,

ledit polymère étant tel que, lorsqu'il est en quantité suffisante dans la composition, la brillance moyenne d'un dépôt de ladite composition, une fois étalée sur un support, est égale ou supérieure à 30.

**7.** Composition cosmétique anhydre sous forme coulée, **caractérisée en ce qu'**elle comprend, dans un milieu physiologiquement acceptable, au moins un polymère de silicone de formule générale (I) :

$$R^1_a R^2_b R^3_c SiO_{(4-a-b-c)/2} \qquad (I).$$

dans laquelle :

- a, b et c sont tels que a varie de 1 à 2,5, b et c, indépendamment l'un de l'autre, varient de 0,001 à 1,5,
- $R^1$ identique ou différent, est choisi parmi :

  - les radicaux alkyle en $C_1$ à $C_{30}$, le cas échéant substitués par un ou plusieurs atomes de fluor, groupements amino et/ou carboxylique,
  - les radicaux aryle, aralkyle,
  - les radicaux de formule générale (II) :

$$-C_dH_{2d}-O-(C_2H_4O)_e(C_3H_6O)_fR^4 \qquad (II)$$

  avec :

    - $R^4$ étant un radical hydrocarboné en $C_1$ à $C_{30}$ ou $R^5$-(CO)-avec $R^5$ étant un radical en $C_1$ à $C_{30}$,
    - d, e et f étant des entiers tels que d varie de 0 à 15, e et f, indépendamment l'un de l'autre, varient de 0 à 50,

  - et leurs combinaisons,

- $R^2$ est un radical de formule générale (III) :

$$-Q-O-X \qquad (III)$$

avec :

  - Q étant un radical hydrocarboné bivalent de $C_2$ à $C_{20}$ pouvant inclure au moins une liaison éther et/ou au moins une liaison ester, et
  - X étant un radical hydrocarboné polyhydroxylé,

- $R^3$ est un organosiloxane de formule générale (IV) :

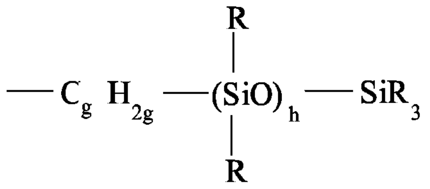

(IV)

avec :

- chacun des groupes R représentant, indépendamment l'un de l'autre, un radical choisi parmi les radicaux alkyle en $C_1$ à $C_{30}$, le cas échéant substitué par un ou plusieurs atomes de fluor, les radicaux aryle, aralkyle,
- g et h étant des entiers tels que g varie de 1 à 5 et h varie de 0 à 500.

**8.** Composition selon l'une quelconque des revendications 1 et 3 à 7, **caractérisée en ce qu'**elle comprend en outre au moins une huile de silicone.

**9.** Composition selon l'une quelconque des revendications précédentes, **caractérisée en ce que** le polymère de silicone est un composé de formule générale (I) dans laquelle $R_1$ est un radical alkyle en $C_1$ à $C_{10}$, en particulier de $C_1$ à $C_6$, et en particulier en $C_1$ à $C_4$.

**10.** Composition selon l'une quelconque des revendications précédentes, **caractérisée en ce que** le polymère de silicone est un composé de formule générale (I) dans laquelle :

- a varie de 1 à 1,4, b et c, indépendamment l'un de l'autre, varient de 0,02 à 0,04,
- $R^1$ est un radical alkyle en $C_1$ à $C_{10}$, en particulier de $C_1$ à $C_6$, et en particulier en $C_1$ à $C_4$,
- $R^2$ est représenté par la formule (IIIA) :

$$-C_3H_6O[CH_2CH(OH)CH_2O]_nH \qquad (IIIA)$$

dans laquelle n varie de 1 à 5, et
- $R^3$ est représenté par la formule (IVA) :

$$-C_2H_4(CH_3)_2SiO[(CH_3)_2SiO]_mSi(CH_3)_3 \qquad (IVA)$$

dans laquelle m varie de 3 à 9.

**11.** Composition selon la revendication précédente, **caractérisée en ce que** le polymère de silicone est un composé de formule générale (I) :

$$R^1{}_a\ R^2{}_b\ R^3{}_c\ SiO_{(4-a-b-c)/2} \qquad (I)$$

dans laquelle :

- a varie de 1 à 1,4, b et c, indépendamment l'un de l'autre, varient de 0,02 à 0,04,
- $R^1$ est un radical méthyle,
- $R^2$ est représenté par la formule (IIIA) dans laquelle n varie de 1 à 5, et
- $R^3$ est représenté par la formule (IVA) dans laquelle m varie de 3 à 9.

**12.** Composition selon l'une quelconque des revendications 2 et 8 à 11, **caractérisée en ce que** l'huile de silicone est choisie parmi :

- les huiles de silicones linéaires ou cycliques, notamment celles ayant une viscosité ≤ 8 centistokes ($8\ 10^{-6}$ $m^2/s$) et en particulier les diméthicones, l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane, et
- les huiles de silicone telles que les polydiméthylsiloxanes (PDMS) non volatiles, les polydiméthylsiloxanes comportant des groupements alkyle ou alcoxy, pendant et/ou en bouts de chaîne siliconée, groupements ayant

chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates, et
- leur mélanges.

**13.** Composition selon l'une quelconque des revendications précédentes, **caractérisée en ce qu'**elle comprend en outre au moins un polyol choisi parmi la glycérine, le propylène glycol, le butylène glycol, le pentylène glycol, l'hexylène glycol, le dipropylène glycol, le diéthylène glycol, le sorbitol, l'hydroxypropyl sorbitol, le 1,2,6-hexanetriol, les éthers de glycol (ayant notamment de 3 à 16 atomes de carbone) tels que les alkyl ($C_1$-$C_4$)éther de mono, di- ou tripropylène glycol, les alkyl($C_1$-$C_4$)éthers de mono, di- ou triéthylène glycol, et leurs mélanges.

**14.** Composition selon l'une quelconque des revendications précédentes, **caractérisée en ce qu'**elle possède une valeur de transfert égale ou inférieure à 45, en particulier égale ou inférieure à 40, notamment égale ou inférieure à 35.

**15.** Composition selon l'une quelconque des revendications précédentes, **caractérisée en ce qu'**elle possède une tenue de la couleur à l'eau égale ou inférieure à 15, en particulier égale ou inférieure à 10, et plus particulièrement égale ou inférieure à 6.

**16.** Composition selon l'une quelconque des revendications précédentes, **caractérisée en ce qu'**elle possède une tenue de la couleur à l'huile égale ou inférieure à 25, en particulier inférieure ou égale à 10, et plus particulièrement inférieure ou égale à 8.

**17.** Composition selon l'une quelconque des revendications précédentes, **caractérisée en ce qu'**elle possède une valeur de brillance moyenne égale ou supérieure à 30, en particulier égale ou supérieure à 40, et plus particulièrement égale ou supérieure à 45.

**18.** Composition selon l'une quelconque des revendications précédentes, **caractérisée en ce qu'**elle est sous forme coulée et présente une dureté variant de 50 à 300 g, en particulier de 70 à 250 g, et plus particulièrement de 100 à 230 g.

**19.** Composition selon l'une quelconque des revendications précédente, **caractérisée en ce qu'**elle comprend en outre au moins une matière colorante.

**20.** Composition selon l'une quelconque des revendications précédentes, **caractérisée en ce qu'**elle comprend en outre au moins une charge.

**21.** Composition selon la revendication précédente, **caractérisée en ce qu'**elle comprend en outre au moins une huile.

**22.** Composition selon la revendication 21, **caractérisée en ce que** ladite huile est une huile hydrocarbonée choisie parmi le polyisobutène hydrogéné, l'heptanoate d'isostéaryle, l'isononanoate d'isononyle, le tétrahydroxystéarate/tétraisostéarate de dipentaérythritol, le 2-octyldodécanol, l'isononanoate d'isotridécyle, le malate de diisostéaryle, et leurs mélanges.

**23.** Composition selon l'une quelconque des revendications précédentes, **caractérisée en ce qu'**elle comprend en outre au moins un composé choisi parmi les cires, les corps gras pâteux, et leurs mélanges.

**24.** Composition selon l'une quelconque des revendications précédentes, **caractérisée en ce qu'**il s'agit d'une composition de maquillage et/ou de soin des lèvres et/ou de la peau, en particulier d'un rouge à lèvres.

**25.** Utilisation d'au moins un polymère de silicone de formule générale (I) telle que définie selon l'une quelconque des revendications 2, 9, 10 ou 11 pour la préparation d'une composition cosmétique anhydre présentant une tenue et une brillance moyenne améliorées.

**26.** Utilisation selon la revendication précédente **caractérisée en ce que** ledit polymère de silicone de formule générale (I) est associé à au moins une huile de silicone.

**27.** Utilisation selon la revendication précédente **caractérisée en ce que** ladite huile de silicone est telle que défmie dans la revendication 12.

**28.** Utilisation selon l'une quelconque des revendications 25 à 27, **caractérisée en ce que** ladite composition présente une valeur de transfert égale ou inférieure à 45, en particulier égale ou inférieure à 40, notamment égale ou inférieure à 35.

**29.** Utilisation selon l'une quelconque des revendications 25 à 28, **caractérisée en ce que** ladite composition présente une tenue de la couleur à l'eau égale ou inférieure à 15, en particulier égale ou inférieure à 10, et plus particulièrement égale ou inférieure à 6.

**30.** Utilisation selon l'une quelconque des revendications 25 à 29, **caractérisée en ce que** ladite composition présente une tenue de la couleur à l'huile égale ou inférieure à 25, en particulier égale ou inférieure à 10, notamment égale ou inférieure à 8.

**31.** Utilisation selon l'une quelconque des revendications 25 à 30, **caractérisée en ce que** ladite composition présente une brillance moyenne égale ou supérieure à 30, en particulier égale ou supérieure à 40, et plus particulièrement égale ou supérieure à 45.

**32.** Utilisation selon l'une quelconque des revendications 25 à 31, **caractérisée en ce que** ladite composition cosmétique est sous forme coulée.

Office européen des brevets

# RAPPORT DE RECHERCHE EUROPEENNE

Numéro de la demande
EP 05 30 0591

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| D,X | EP 1 213 316 A (SHIN-ETSU CHEMICAL CO., LTD) 12 juin 2002 (2002-06-12)<br>* alinéas [0001] - [0006], [0018] - [0023], [0101] - [0132]; exemples 12,13 *<br>* exemples 1-3,6-8,11,12,15,16,18-24,26,29 *<br>----- | 1-32 | A61Q1/06<br>A61K8/37<br>A61K8/58<br>A61K8/894 |
| D,X | EP 1 416 016 A (SHIN-ETSU CHEMICAL CO., LTD) 6 mai 2004 (2004-05-06)<br>* alinéas [0012], [0014], [0040]; revendications 1-12,14-37; exemples 25,31,36 *<br>----- | 1-32 | |
| X | DATABASE WPI<br>Section Ch, Week 200356<br>Derwent Publications Ltd., London, GB;<br>Class A14, AN 2003-597917<br>XP002318330<br>& WO 03/041664 A1 (SHINETSU CHEM CO LTD)<br>22 mai 2003 (2003-05-22)<br>* abrégé * | 1-32 | |
| X | & WO 03/041664 A (SHIN-ETSU CHEMICAL CO., LTD; NAKANISHI, TETSUO; TACHIBANA, KIYOMI)<br>22 mai 2003 (2003-05-22)<br>abstract<br>----- | 1-32 | **DOMAINES TECHNIQUES RECHERCHES (IPC)**<br>A61K |
| Y | PATENT ABSTRACTS OF JAPAN<br>vol. 1997, no. 07,<br>31 juillet 1997 (1997-07-31)<br>& JP 09 071504 A (KOSE CORP; SHIN ETSU CHEM CO LTD), 18 mars 1997 (1997-03-18)<br>* abrégé *<br>----- | 1-32 | |
| Y | US 5 306 838 A (SHIOYA ET AL)<br>26 avril 1994 (1994-04-26)<br>* colonne 2, ligne 10 - ligne 69 *<br>-----<br>-/-- | 1-32 | |

Le présent rapport a été établi pour toutes les revendications

2

| Lieu de la recherche | Date d'achèvement de la recherche | Examinateur |
|---|---|---|
| Munich | 12 octobre 2005 | Krattinger, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................
& : membre de la même famille, document correspondant

EPO FORM 1503 03.82 (P04C02)

Office européen des brevets

# RAPPORT DE RECHERCHE EUROPEENNE

Numéro de la demande
EP 05 30 0591

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| P,X | EP 1 496 080 A (SHIN-ETSU CHEMICAL COMPANY, LTD) 12 janvier 2005 (2005-01-12)<br>* exemple 62 *<br>----- | 1-32 | |
| P,X | EP 1 481 660 A (SHIN-ETSU CHEMICAL CO., LTD) 1 décembre 2004 (2004-12-01)<br>* exemples 13,22,43,44,53,55,56,58-60 *<br>* exemples 66,67,69,70 *<br>----- | 1-32 | |
| P,X | DATABASE WPI<br>Section Ch, Week 200477<br>Derwent Publications Ltd., London, GB;<br>Class A26, AN 2004-784492<br>XP002318331<br>& WO 2004/091562 A1 (SHINETSU CHEM CO LTD)<br>28 octobre 2004 (2004-10-28)<br>* abrégé * | 1-32 | |
| P,X | & WO 2004/091562 A (SHIN-ETSU CHEMICAL CO., LTD; KAMEI, MASANAO; TACHIBANA, KIYOMI) 28 octobre 2004 (2004-10-28)<br>abstract<br>----- | 1-32 | DOMAINES TECHNIQUES RECHERCHES (IPC) |
| P,X | DATABASE WPI<br>Section Ch, Week 200505<br>Derwent Publications Ltd., London, GB;<br>Class A26, AN 2005-047910<br>XP002318332<br>& WO 2004/100916 A1 (KOSE CORP)<br>25 novembre 2004 (2004-11-25)<br>* abrégé * | 1-32 | |
| P,X | & WO 2004/100916 A (KOSE CORPORATION; WATANABE, SOICHIRO)<br>25 novembre 2004 (2004-11-25)<br>abstract<br>----- | 1-32 | |
| D,X | EP 1 424 373 A (SHIN-ETSU CHEMICAL COMPANY, LTD) 2 juin 2004 (2004-06-02)<br>* exemples 1-4 *<br>----- | 1-32 | |

2

Le présent rapport a été établi pour toutes les revendications

| Lieu de la recherche | Date d'achèvement de la recherche | Examinateur |
|---|---|---|
| Munich | 12 octobre 2005 | Krattinger, B |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 05 30 0591

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

12-10-2005

| Document brevet cité au rapport de recherche | | Date de publication | Membre(s) de la famille de brevet(s) | | Date de publication |
|---|---|---|---|---|---|
| EP 1213316 | A | 12-06-2002 | JP | 2002179798 A | 26-06-2002 |
| | | | US | 2002131947 A1 | 19-09-2002 |
| EP 1416016 | A | 06-05-2004 | US | 2004091439 A1 | 13-05-2004 |
| WO 03041664 | A1 | 22-05-2003 | JP | 3678420 B2 | 03-08-2005 |
| WO 03041664 | A | 22-05-2003 | JP | 3678420 B2 | 03-08-2005 |
| JP 09071504 | A | 18-03-1997 | JP | 3513682 B2 | 31-03-2004 |
| US 5306838 | A | 26-04-1994 | AUCUN | | |
| EP 1496080 | A | 12-01-2005 | JP | 2005042097 A | 17-02-2005 |
| | | | US | 2005008600 A1 | 13-01-2005 |
| EP 1481660 | A | 01-12-2004 | JP | 2004346046 A | 09-12-2004 |
| | | | US | 2004241126 A1 | 02-12-2004 |
| WO 2004091562 | A1 | 28-10-2004 | AU | 2003236256 A1 | 04-11-2004 |
| WO 2004091562 | A | 28-10-2004 | AU | 2003236256 A1 | 04-11-2004 |
| WO 2004100916 | A1 | 25-11-2004 | AUCUN | | |
| WO 2004100916 | A | 25-11-2004 | AUCUN | | |
| EP 1424373 | A | 02-06-2004 | JP | 2004155978 A | 03-06-2004 |
| | | | US | 2004091440 A1 | 13-05-2004 |

EPO FORM P0460

Pour tout renseignement concernant cette annexe : voir Journal Officiel de l'Office européen des brevets, No.12/82